(19) Europäisches Patentamt
European Patent Office
Office européen des brevets

(11) EP 4 530 343 A1

(12) EUROPEAN PATENT APPLICATION

(43) Date of publication:
02.04.2025 Bulletin 2025/14

(21) Application number: 24197181.1

(22) Date of filing: 29.08.2024

(51) International Patent Classification (IPC):
C12N 1/20 (2006.01)    C12R 1/01 (2006.01)
H01M 8/16 (2006.01)

(52) Cooperative Patent Classification (CPC):
C12N 1/205; H01M 8/16; C12R 2001/01

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
GE KH MA MD TN

(30) Priority: 01.09.2023  JP 2023142590
24.11.2023  JP 2023199255
19.01.2024  JP 2024006867

(71) Applicants:
• Tokyo University of Pharmacy and Life Science
Hachioji-shi, Tokyo 192-0392 (JP)
• Seiko Epson Corporation
Tokyo 160-8801 (JP)

(72) Inventors:
• YAMADA, Yohei
Nagano, 392-8502 (JP)
• KAMAKURA, Tomoyuki
Nagano, 392-8502 (JP)
• HARADA, Tomoka
Tokyo, 192-0392 (JP)
• WATANABE, Kazuya
Tokyo, 192-0392 (JP)

(74) Representative: Becker, Eberhard
Becker Kurig & Partner
Patentanwälte mbB
Bavariastraße 7
80336 München (DE)

(54) MICROORGANISM, MICROBIAL FUEL CELL COMPOSITION, MICROBIAL FUEL CELL, MICROBIAL ELECTROLYSIS CELL COMPOSITION, AND MICROBIAL ELECTROLYSIS CELL

(57) Provided are a microorganism which is a Geobacter sp. strain 60473; a microbial fuel cell composition containing the Geobacter sp. strain 60473 and an organic substance; a microbial fuel cell in which the Geobacter sp. strain 60473 is used to decompose the organic substance as a fuel; a microbial electrolysis cell composition containing the Geobacter sp. strain 60473 and the organic substance; and a microbial electrolysis cell in which the Geobacter sp. strain 60473 is used to decompose the organic substance as the fuel.

FIG. 1

EP 4 530 343 A1

**Description**

The present application is based on, and claims priority from JP Application Serial Number 2023-142590, filed on September 1, 2023; JP Application Serial Number 2023-199255, filed on November 24, 2023; and JP Application Serial Number 2024-006867, filed on January 19, 2024, the disclosure of which is hereby incorporated by reference herein in its entirety.

BACKGROUND

1. Technical Field

The present disclosure relates to a microorganism used in a microbial fuel cell, and a microbial fuel cell using the microorganism.

2. Related Art

In order to assist in solving problems such as global warming and depletion of natural resources, a bioelectrochemical system (BES), which is a system for electrochemically controlling metabolism of microorganisms to produce energy and useful substances, has been actively developed. Examples of the microorganisms used in the bioelectrochemical system include electrochemically active bacteria (EAB) capable of exchanging electrons with an electrode.

Examples of the bioelectrochemical system include a microbial fuel cell (MFC), a microbial electrolysis cell (MEC), and a microbial electrosynthesis system (MES). The microbial fuel cell is a device that converts a fuel (a substance retaining chemical energy) such as an organic substance into electric energy by utilizing a metabolism ability of a microorganism. The microbial fuel cell is attracting attention as a device that generates power while processing biomass waste and as energy-saving wastewater treatment (Shohei Yamada and Kazuya Watanabe, "Basics and Applications of Microbial Fuel Cells", Fuel Cells, Fuel Cell Development Information Center (Publisher), 2020, vol. 20, pp. 33-38.). The microbial electrolysis cell is a device that generates hydrogen and methane by reducing protons in water using a current generated from fuel such as an organic substance by electrochemically active bacteria (Ichita Ochiai et al., "Hydrogen Production Using Microbial Electrolysis Tank", Power Generation and Hydrogen Production Using Microorganisms, CMC Publishing Co., Ltd. (published), 2021, pp. 201-206.). The microbial electrosynthesis system is a device that synthesizes useful substances from carbon dioxide, nitrogen, and the like by supplying electrons to electrochemically active bacteria from a low-potential electrode, and is expected that the processing will support humanity in the future when fossil fuels are depleted (Yamada et al., Frontiers in Chemistry, 2022, vol. 9, Article 805597.). As described above, various devices are included in the bioelectrochemical system, and high performance is desired for social implementation.

In each device of the bioelectrochemical system, it is said that electron transfer efficiency between the electrochemically active bacteria and the electrode, which is evaluated by a current value per electrode area (current density), determines device performance. In fact, it is reported that exchange of electrons between the electrochemically active bacteria and the electrode is a rate-limiting step in the bioelectrochemical system (Lu and Ren, Bioresource Technology, 2016, vol. 215, p. 254-264.).

As a method to enhance activity (power generation capacity) of the electrochemically active bacteria, a known method (bioaugmentation) is to introduce artificially cultured foreign microorganisms (for example, electrochemically active bacteria) into a microbial ecosystem within the device as a microbial preparation to improve the device performance. For example, a case is reported in which rise of current generation is accelerated by introducing a microbial consortium containing electrochemically active bacteria into a microbial fuel cell for wastewater treatment (Yanuka-Golub et al., mBio, 2021, vol. 12, e03629-20.). However, in the example, a current value after the current rises is equivalent to that of a microbial fuel cell without bioaugmentation, and further improvement in performance is desired. In addition, it is known that the current generation and the hydrogen production are promoted by bioaugmentation in which a Geobacter sulfur reduction strain YM18 is added to an anode electrode of a microbial fuel cell (Ochiai et al., Bioresource Technology, 2023, vol. 386, 129508).

As a method to increase a power generation amount (output energy) of a microbial fuel cell, there is a method using, for example, a compound that transfers electrons (electron mediator) such as HNQ (2-hydroxy-1,4-naphthoquinone), and as an improved method of the method, a method is reported that uses electrochemically active bacteria that are active even in an environment without light and uses porphyrin as the electron mediator (JP-A-2010-218690). In order to enhance activity of the electrochemically active bacteria, a method is reported in which the activity of electrochemically active bacteria is adjusted using a bacteriophage that uses the electrochemically active bacteria as a host (JP-A-2020-14418). However, regarding the use of mediators, since the mediators disappear due to decomposition or the like by the microorganisms, it is necessary to continuously add the mediators artificially, and cost may increase in practical terms. In addition, regarding the use of bacteriophages, certification to check that the phage does not contain genes involved in pathogenicity and drug resistance may be costly.

In order to improve performance of each device in a bioelectrochemical system, electrochemically active bacteria that can achieve high current density are desired. For example, in an example of bioaugmentation reported in Lu and Ren, Bioresource Technology, 2016, vol. 215, p. 254-264., although it has not yet been possible to sufficiently increase the activity of electrochemically active bacteria in the bioelectrochemical system, this is thought to be because electrochemically active bacteria with sufficiently high electrochemical activity are not introduced. In a strain YM16 used in the microbial fuel cell reported in Ochiai et al., Bioresource Technology, 2023, vol. 386, 129508, an amount of generated current decreases as a power generation time increases. Therefore, there is a need for electrochemically active bacteria that can generate stable power for a longer period of time.

An object of the present disclosure is to provide a novel microorganism having higher current generating ability, a microbial fuel cell composition and a microbial electrolysis cell composition containing the microorganism, and a microbial fuel cell and a microbial electrolysis cell using the microorganism.

SUMMARY

As a result of intensive studies to solve the above problems, an inventor of the present disclosure has found that a novel microorganism isolated and identified from mud on the shores of Lake Suwa is a novel electrochemically active bacterium that has a high current generating ability and is suitable for use in a microbial fuel cell and a microbial electrolysis cell, leading to completion of the present disclosure.

A microorganism, a microbial fuel cell composition, a microbial fuel cell, a microbial electrolysis cell composition, and a microbial electrolysis cell according to the present disclosure are as follows.

[1] A microorganism contains: a Geobacter sp. strain 60473 (accession No.: NITE BP-03900).

[2] The microorganism according to [1] is used in a microbial fuel cell.

[3] A microbial fuel cell composition contains: a Geobacter sp. strain 60473 (accession No.: NITE BP-03900); and an organic substance.

[4] The microbial fuel cell composition according to [3] further contains: an electrochemically active bacterium other than the Geobacter sp. strain 60473.

[5] In the microbial fuel cell composition according to [3] or [4], at least a part of the organic substance is sludge.

[6] In a microbial fuel cell, a Geobacter sp. strain 60473 (accession No.: NITE BP-03900) is used to decompose an organic substance as a fuel.

[7] A microbial fuel cell contains:

a liquid containing water containing an organic substance as a main component;
a container configured to contain the liquid;
a conductive anode electrode disposed in the liquid;
a conductive cathode electrode disposed with at least one surface thereof in contact with the liquid;
a resistor having one end connected to the anode electrode and the other end connected to the cathode electrode, through which electrons flow; and
a Geobacter sp. strain 60473 (accession No.: NITE BP-03900), which decomposes the organic substance contained in the liquid and discharges the electrons.

[8] In the microbial fuel cell according to [7], the container is divided, by a membrane that allows protons to be selectively transmitted, into a tank in which the anode electrode is disposed and a tank in which the cathode electrode is disposed, and
the organic substance and the Geobacter sp. strain 60473 are contained in the liquid contained in the tank in which the anode electrode is disposed.

[9] In the microbial fuel cell according to [7], the cathode electrode is in contact with the liquid in the container on one surface and in contact with an atmosphere on the other surface.

[10] A microbial fuel cell contains:

a container containing organic mud and a liquid containing water as a main component;
a conductive anode electrode disposed in the organic mud;
a conductive cathode electrode disposed within the liquid or on a liquid surface of the liquid;
a resistor having one end connected to the anode electrode and the other end connected to the cathode electrode, through which electrons flow; and
a Geobacter sp. strain 60473 (accession No.: NITE BP-03900) contained in the organic mud.

[11] A microbial fuel cell to be installed to purify organic mud at a bottom of a river or a lake, the microbial fuel cell contains:

a conductive anode electrode disposed in organic mud;
a conductive cathode electrode disposed in water or on a water surface;
a resistor having one end connected to the anode electrode and the other end connected to the cathode electrode, through which electrons flow; and
a Geobacter sp. strain 60473 (accession No.: NITE BP-03900) adhering to the anode electrode.

[12] The microbial fuel cell according to [10] or [11] further contains: a supply tube configured to supply a substrate and/or the Geobacter sp. strain 60473 into the organic mud from outside.

[13] In the microbial fuel cell according to [12], the supply tube is branched into a plurality of tubes in a dispersed manner in a planar direction of the anode electrode so as to enable a supply into the liquid or the water.

[14] In the microbial fuel cell according to any one of [7], [10], and [11], the anode electrode is a conductive metal material having an internal electrical resistance lower than an internal electrical resistance of the cathode electrode.

[15] In the microbial fuel cell according to any one of [7], [10], and [11], a surface area of the cathode electrode is larger than a surface area of the anode electrode by 1.1 times or more.

[16] In the microbial fuel cell according to any one of [10] and [11], the cathode electrode and the anode electrode are disposed in parallel, one end of the cathode electrode and one end of the anode electrode disposed on a same side as the one end are connected to a support body extending in a depth direction of the liquid or the water, the cathode electrode and the anode electrode are fixedly supported in an adjustable manner while maintaining an interval corresponding to a depth, and an installation fixing portion that protrudes in the depth direction is provided on a bottom side of the anode electrode.

[17] A microbial electrolysis cell composition contains: a Geobacter sp. strain 60473 (accession No.: NITE BP-03900); and an organic substance.

[18] The microbial electrolysis cell composition according to [17] further contains: an electrochemically active bacterium other than the Geobacter sp. strain 60473.

[19] In the microbial electrolysis cell composition according to [17] or [18], at least a part of the organic substance is sludge.

[20] In a microbial electrolysis cell, a Geobacter sp. strain 60473 (accession No.: NITE BP-03900) is used to decompose an organic substance as a fuel.

[21] A microbial electrolysis cell contains:

a liquid containing water containing an organic substance as a main component;
a container configured to contain the liquid;
a conductive anode electrode disposed in the liquid;
a conductive cathode electrode disposed in contact with the liquid;
a potential control device having one end connected to the anode electrode and the other end connected to the cathode electrode, through which electrons flow; and
a Geobacter sp. strain 60473 (accession No.: NITE BP-03900) which decomposes the organic substance contained in the liquid and discharges the electrons.

[22] In the microbial electrolysis cell according to [21], the container is divided, by a membrane that allows protons to be selectively transmitted, into a tank in which the anode electrode is disposed and a tank in which the cathode electrode is disposed, and
the organic substance and the Geobacter sp. strain 60473 are contained in the liquid contained in the tank in which the anode electrode is disposed.

[23] A microbial electrolysis cell contains:

a container containing organic mud and a liquid containing water as a main component;
a conductive anode electrode disposed in the liquid;
a conductive cathode electrode disposed in the liquid;
a potential control device having one end connected to the anode electrode and the other end connected to the cathode electrode, through which electrons flow; and
a Geobacter sp. strain 60473 (NITE BP-03900) contained in the organic mud.

[24] The microbial electrolysis cell according to [23] further contains: a supply tube configured to supply a substrate and/or the Geobacter sp. strain 60473 into the liquid from outside.

[25] In the microbial electrolysis cell according to [24], the supply tube is branched into a plurality of tubes in a dispersed manner in the anode electrode or the cathode electrode so as to enable a supply into the liquid.

[26] In the microbial electrolysis cell according to any one of [21], [23], and [24], the anode electrode is a conductive material having an internal electrical resistance lower than an internal electrical resistance of the cathode electrode.

[27] In the microbial electrolysis cell according to any one of [21], [23], and [24], a surface area of the cathode electrode is larger than a surface area of the anode electrode by 1.1 times or more.

According to the present disclosure, it is possible to provide a microorganism having a fairly high current generating ability and a hydrogen producing ability. By using the microorganism, it is possible to provide a microbial fuel cell having good power generation efficiency and a microbial electrolysis cell having good electrolysis efficiency.

BRIEF DESCRIPTION OF THE DRAWINGS

FIG. 1 is a schematic view of one embodiment of a microbial fuel cell in which a microbial supply device is installed.

FIG. 2 is a schematic view of one embodiment of a microbial fuel cell provided with a support device that facilitates a movement.

FIG. 3 is a schematic view of one embodiment of a three-electrode single-tank microbial electrolysis cell.

FIG. 4 is a schematic view of a structure of a mud cell used to isolate a strain 60473 in Example 1.

FIG. 5 is a diagram showing results obtained by measuring a current generated by planting the strain 60473 and a current generated by planting a strain PCA over time in an electrochemical cell using a graphite felt electrode as a working electrode in Example 1.

FIG. 6 is a diagram showing results obtained by measuring the current generated by planting the strain 60473 and the current generated by planting the strain PCA over time in the electrochemical cell using a glassy carbon electrode as the working electrode in Example 1.

FIG. 7 is a diagram showing results obtained by measuring a fluorescence intensity by DAPI staining of the glassy carbon electrode after completion of a current generation experiment in Example 1.

FIG. 8 is a diagram showing a phylogenetic relationship between the strain 60473 and closely related strains, as shown by molecular phylogenetic analysis using a 16S rRNA gene in Example 1.

FIG. 9 is a diagram showing the phylogenetic relationship between the strain 60473 and the closely related strains, as shown by the molecular phylogenetic analysis using a gyrB gene in Example 1.

FIG. 10 is a diagram showing polarization analysis results of the mud cell into which the strain 60473 is introduced in Example 2.

FIG. 11 is a diagram showing polarization analysis results of the mud cell into which the strain 60473 is not introduced in Example 2.

FIG. 12 is a diagram showing a maximum power density ($P_{max}$ mW/m$^2$) calculated based on the polarization analysis results shown in FIGS. 10 and 11 in Example 2.

FIG. 13 is a diagram showing results obtained by measuring currents generated by planting the strain 60473, the strain PCA, a strain KN400, or a strain YM18 over time in the electrochemical cell using the graphite felt electrode as the working electrode in Example 3.

FIG. 14 is a diagram showing results obtained by measuring maximum current densities of the currents generated by planting the strain 60473, the strain PCA, the strain KN400, or the strain YM18 in the electrochemical cell using the graphite felt electrode as the working electrode in Example 3.

FIG. 15 is a diagram showing results obtained by measuring currents generated by planting the strain 60473, the strain PCA, the strain KN400, or the strain YM18 over time in the electrochemical cell using the glassy carbon electrode as the working electrode in Example 3.

FIG. 16 is a diagram showing results obtained by measuring maximum current densities of the currents generated by planting the strain 60473, the strain PCA, the strain KN400, or the strain YM18 in the electrochemical cell using the glassy carbon electrode as the working electrode in Example 3.

FIG. 17 is a diagram showing measurement results of graphite affinity (GAI) of the strain 60473, the strain PCA, the strain KN400, and the strain YM18 in Example 3.

FIG. 18 is a diagram showing measurement results of cell hydrophobicity (HPI) of the strain 60473, the strain PCA, the strain KN400, and the strain YM18 in Example 3.

FIG. 19 is a diagram showing measurement results of cytochrome contents (CCI) of the strain 60473, the strain PCA, the strain KN400, and the strain YM18 in Example 3.

FIG. 20 is a diagram showing results obtained by measuring currents generated over time in electrolysis cells (MEC3 and MEC4) planted with the strain 60473 and electrolysis cells (MEC1 and MEC2) not planted with the strain 60473 in Example 4.

FIG. 21 is an enlarged diagram showing the results obtained by measuring the currents generated over time in the electrolysis cells (MEC1 and MEC2) not planted with the strain 60473 in Example 4.

FIG. 22 is a diagram showing a bacterial composition of a biofilm formed on a surface of the working electrode (anode electrode) after an operation of the electrolysis cells (MEC3 and MEC4) planted with the strain 60473 and the electrolysis cells (MEC1 and MEC2) not planted with the strain 60473 in Example 4.

FIG. 23 is a diagram showing results obtained by measuring currents generated over time in electrolysis cells (60473_1 and 60473_2) planted with the strain 60473 and electrolysis cells (NB_1 and NB_2) not planted with the strain 60473 in Example 5.

FIG. 24 is a current-voltage curve obtained by cyclic voltammetry (CV) of the electrolysis cells (60473_1 and 60473_2) planted with the strain 60473 and the electrolysis cells (NB_1 and NB_2) not planted with the strain 60473 in Example 5.

FIG. 25 is a diagram showing results obtained by measuring a voltage (V) of each reactor over time in Example 6.

FIG. 26 is a diagram showing polarization analysis results of MFC reactors (60473_1 and 60473_2) into which the strain 60473 is introduced in Example 6.

FIG. 27 is a diagram showing polarization analysis results of MFC reactors (NB_1 and NB_2) into which the strain 60473 is not introduced in Example 6.

FIG. 28 is a diagram showing a maximum power density ($P_{max}$ mW/m$^2$) calculated based on the polarization analysis results shown in FIGS. 26 and 27 in Example 6.

FIG. 29 is a current-voltage curve obtained by the CV of the MFC reactors (60473_1 and 60473_2) planted with the strain 60473 and the MFC reactors (NB_1 and NB_2) not planted with the strain 60473 in Example 6.

DESCRIPTION OF EMBODIMENTS

In the present disclosure and the present specification, "$X_1$ to $X_2$ ($X_1$ and $X_2$ are natural numbers satisfying $X_1 < X_2$)" means a numerical range of "$X_1$ or more and $X_2$ or less".

Microorganism for Microbial Fuel Cell

A microorganism according to one embodiment of the present disclosure is a Geobacter sp. strain 60473 (Geobacter sp. 60473) (hereinafter, sometimes referred to as a "strain 60473"). The strain 60473 is a novel electrochemically active bacterium isolated and identified from mud on the shores of Lake Suwa, which decomposes an organic substance and releases electrons. As shown in Example 1 below, based on results of genome analysis, the closest known relative of the strain 60473 is a Geobacter sulfurreducens strain PCA (hereinafter, referred to as a "strain PCA"), which is a reference strain of Geobacter sulfurreducens, which is commonly used as an electrochemically active bacterium. The inventors of the present disclosure deposits the strain 60473 at the Patent Microorganism Depositary Center of the National Institute of Technology and Evaluation (Room 122, 2-5-8 Kazusa Kamatari, Kisarazu City, Chiba Prefecture). An accession number of the Geobacter sp. strain 60473 is NITE BP-03900 (deposit date: June 8, 2023).

The strain 60473 is a Gram-negative anaerobic bacillus with flagella, and is a type of iron-reducing bacterium that obtains energy by reducing iron (III) ions and oxidizing acetic acid to carbon dioxide. The strain 60473 can be cultured in the same manner as other Geobacter bacteria. For example, the strain 60473 can be cultured at 10°C to 40°C in an anaerobic environment in a medium containing necessary minerals and vitamins. The anaerobic environment in which the strain 60473 is cultured is not particularly limited as long as an oxygen gas concentration is 0.01% (v/v) or less. The strain 60473 can be cultured, for example, in an environment where a nitrogen gas concentration is controlled to be 99.99% (v/v) or higher, and preferably 99.999%. Cultivation in such an anaerobic environment can be carried out by using a culture system for anaerobic bacteria such as AneroPack Pouch (manufactured by Mitsubishi Gas Chemical Co., Ltd.).

The medium used for culturing the strain 60473 only needs to contain the necessary minerals and vitamins, and various media commonly used for culturing Geobacter bacteria including Geobacter sulfluducens, such as a DSM826 medium (manufactured by DSMZ), and modified media thereof can be used. For culturing the strain 60473, a liquid medium or an agar medium may be used. Since the Geobacter bacteria are soil bacteria, the strain 60473 can also be cultured in soil or sludge. The strain 60473 can be cultured even in an anaerobic environment rich in an organic substance.

The strain 60473 has fairly high power generation capacity, and its current generating ability in the electrochemical cell is higher than the strain PCA. Therefore, by using the strain 60473 as an electrochemically active bacterium, a microbial fuel cell with high power generation capacity can be produced. Further, the strain 60473, like other electrochemically active bacteria, is also useful as an electrochemically active bacterium used in bioelectrochemical systems other than the microbial fuel cell, such as a microbial electrolysis cell and a microbial electrosynthesis system.

The strain 60473 has fairly high graphite affinity (GAI). Therefore, the strain 60473 is suitable for biofilm formation on graphite surfaces, and is particularly suitable as an electrochemically active bacterium used in a microbial fuel cell and a microbial electrolysis cell that use a conductive carbon-based anode electrode containing carbon or graphite as a main component. The GAI of the strain 60473 is 60% to 80%, and preferably 65% to 80%. The GAI of microorganisms including the strain 60473 can be measured by a method shown in Examples below.

The strain 60473 has high cell hydrophobicity (HPI). Therefore, the strain 60473 has good adhesion to a hydrophobic electrode such as a carbon-based electrode, and it is easy to form a biofilm on a surface of the hydrophobic electrode. The HPI of the strain 60473 is 50% to 90%, preferably 60% to 90%, and more preferably 65% to 90%. The HPI of microorganisms including the strain 60473 can be measured by a method shown in Examples below.

The strain 60473 has a high cytochrome content (CCI) per protein (mass). Cytochrome is a general term for proteins that have redox-capable iron-bound heme (containing iron atoms in the form of heme groups), and is broadly classified into cytochromes a, b, c, d, and the like depending on a type of heme and a binding mode. Since the cytochrome is a molecule related to extracellular electron transport ability, the high CCI is presumed to be one reason for excellent power generation capacity of the strain 60473. The CCI of the strain 60473 is 0.75/mg to 1.00/mg. The CCI of microorganisms including the strain 60473 can be measured by a method shown in Examples below.

When using the strain 60473 as an electrochemically active bacterium in a microbial fuel cell, it may be used alone, and it may be used in combination with other electrochemically active bacteria, and it is also preferable to use it in combination with other microorganisms other than the electrochemically active bacteria. For example, by using anaerobic bacteria having an acetic acid producing ability, acetic acid that decreases due to growth of the strain 60473 can be supplied, so that a culture period during which the strain 60473 can exert its power generation capacity can be extended.

The strain 60473 is also suitable as an active ingredient of a microbial preparation added to a microbial fuel cell by a bioaugmentation method. The bioaugmentation microbial preparation may be a culture of the strain 60473, a fungus body obtained by centrifuging and precipitating the culture, or a composition obtained by mixing the culture with other additives (for example, an excipient) as necessary, and the composition may be further formed into structured granules. The bioaugmentation microbiological preparation may be a suspension-like composition obtained by suspending the fungus bodies of the strain 60473 in water, a culture medium, or the like, and further containing other components as necessary. The bioaugmentation microbial preparation may contain one or more types of microorganisms other than the strain 60473. Examples of the microorganisms include anaerobic acetic acid-producing bacteria.

Microbial Fuel Cell Composition

A microbial fuel cell composition in the embodiment contains the strain 60473 and an organic substance, and is a composition that is used as an organic substance serving as a fuel for a microbial fuel cell or is added to the fuel. The microbial fuel cell composition can also be used as the bioaugmentation microbial preparation. A form of the microbial fuel cell composition in the embodiment is not particularly limited, and may be a solid substance such as a granule or a lump, a paste, a gel, or a suspension.

The organic substance contained in the microbial fuel cell composition in the embodiment is not particularly limited as long as organic substance serves as the fuel for the microbial fuel cell. Biomass is preferably used, a chemically synthesized organic substance, a fossil fuel, or a processed product thereof may also be used. As the organic substance contained in the microbial fuel cell composition in the embodiment, organic substances derived from waste biomass such as food waste, agricultural residue, livestock manure, sewage sludge, bottom mud of rivers and lakes (organic mud at the bottom), wood waste, paper waste, and wastewater are particularly preferable. The organic substance contained in the microbial fuel cell composition may be a mixture of organic substances derived from a plurality of biomass.

The microbial fuel cell composition in the embodiment may contain one or more types of microorganisms other than the strain 60473. The microorganisms may be electrochemically active bacteria or may be other than the electrochemically active bacteria. Any microorganism can be used as long as it can grow in an environment where the strain 60473 can grow, and is preferably an anaerobic bacterium, and may also be a microaerobic bacterium. Examples of the microorganisms other than the strain 60473 contained in the microbial fuel cell composition include electrochemically active bacteria and anaerobic acetic acid-producing bacteria.

Microbial Fuel Cell

The microbial fuel cell in the embodiment is characterized in that the strain 60473 is used for decomposition of an organic substance as a fuel. In the microbial fuel cell according to the embodiment, the microorganism used for the decomposition of the organic substance may be only the strain 60473, or the strain 60473 may be used in combination with other microorganisms. By using the strain 60473 and the anaerobic acetic acid-producing bacteria in combination, acetic acid essential for power generation by the strain 60473 is stably supplied, and the power generation by the strain 60473 can be stably performed for a longer period of time. By using a wide variety of microorganisms capable of decomposing the organic substance in combination, the organic substance serving as the fuel can be efficiently decomposed into a state that can be easily used by the strain 60473. It is also preferable to use the electrochemically active bacteria other than the strain 60473 in combination because it can enhance power generation efficiency for the organic substance serving as the fuel.

In the microbial fuel cell in the embodiment, an amount of the strain 60473 to be used is not particularly limited, and is appropriately adjusted in consideration of a type and a concentration of the organic substance used as the fuel, a type of anode electrode, presence or absence of adhesion to the anode electrode, and the like. When the strain 60473 is dispersed in a liquid containing the organic substance, the amount of the strain 60473 per gram of the liquid can be, for example, 10,000 cells/g to 100,000,000 cells/g, and is preferably 100,000 cells/g to 10,000,000 cells/g. When the strain 60473 adheres to the anode electrode, the amount of the strain 60473 per 1 $cm^2$ of a surface area of the anode electrode can be, for example, 10,000 cells/g to 100,000,000 cells/g, and is preferably 100,000 cells/g to 10,000,000 cells/g.

In the microbial fuel cell in the embodiment, when using the strain 60473 and other electrochemically active bacteria in combination, a ratio of the strain 60473 to a total amount of the electrochemically active bacteria in the microbial fuel cell is not particularly limited, and is preferably 1 mass% or more, more preferably 10 mass% or more, and even more preferably 30 mass% or more. Since the strain 60473 has high power generation capacity, even when a small amount is added to a microbial fuel cell using the electrochemically active bacteria in the related art, an effect of increasing a power generation amount can be expected.

The power generation capacity of the strain 60473 decreases as a cell usage time increases. Therefore, in the microbial fuel cell according to the embodiment, it is preferable to newly supply the strain 60473 at a timing when the power generation capacity of the strain 60473 decreases after the start of the operation of the cell. In addition to the supply of the strain 60473, anaerobic acetic acid-producing bacteria and other microorganisms can also be supplied.

The timing to newly supply the strain 60473 can be determined, for example, by monitoring a transition of a generated voltage by the microbial fuel cell. In the microbial fuel cell, the generated voltage becomes stable after a while after the start of operation. Based on a generated voltage value in a state in which the generated voltage is stable (stable generated voltage value), when the generated voltage value falls below a preset threshold value (for example, a 70% value of the stable generated voltage value), the strain 60473 is newly supplied, so that the power generation can be stably performed for a long period of time. The new strain 60473 may be supplied every predetermined time from the start of the operation of the cell.

The microbial fuel cell in the embodiment uses the strain 60473, and only needs to include an anode electrode, a cathode electrode, a resistor connected to both electrodes, through which electrons flow, and an organic substance serving as a fuel, which are essential components of the cell, and other structures are not particularly limited. Specifically, the microbial fuel cell according to the embodiment includes a liquid containing water containing an organic substance as a main component, a container that contains the liquid, a conductive anode electrode disposed in the liquid, a conductive cathode electrode disposed with at least one surface thereof in contact with the liquid, and a resistor having one end connected to the anode electrode and the other end connected to the cathode electrode, through which electrons flow. Microorganisms including the strain 60473 are contained in the liquid in which the anode electrode is disposed. Examples of the organic substance serving as the fuel include the organic substances same as those contained in the microbial fuel cell composition. In the microbial fuel cell in the embodiment, it is preferable to use an organic substance containing phosphorus, nitrogen, and sulfide.

On an anode electrode side, the organic substance serving as the fuel is decomposed by the microorganisms to generate carbon dioxide, and during the reaction, the electrochemically active bacteria such as the strain 60473 emit electrons. The anode electrode is not particularly limited as long as it is an electrode made of a conductive material capable of receiving electrons produced from electrochemically active bacteria and delivering the electrons to the cathode electrode through the resistor. In the microbial fuel cell according to the embodiment, a conductive carbon-based anode electrode containing carbon or graphite as a main component is preferable from the viewpoint of good adhesion of microorganisms, and an anode electrode made of a carbon cloth (a cloth made by spinning carbonized fibers obtained by firing petroleum or acrylic fibers) is more preferable from the viewpoint of a large surface area.

The strain 60473 tends to adhere to a smooth surface, and can adhere to a metal surface. In addition, a metal electrode has a lower internal electric resistance than a graphite electrode, and can prevent a decrease in power generation efficiency. Therefore, in the microbial fuel cell according to the embodiment, it is also preferable that the anode electrode is made of a conductive metal material with a lower internal electrical resistance than the cathode electrode. By using the anode electrode made of metal such as stainless steel, iron, or magnesium and having the strain 60473 adhering to a surface thereof, a microbial fuel cell having more excellent power generation efficiency can be obtained.

In the cathode electrode, a reduction reaction is caused by the electrons received from the anode electrode through the resistor, a compound serving as an oxidant (electron acceptor), and protons diffused from the anode electrode. As the cathode electrode, an electrode in which a catalyst for a reduction reaction such as platinum is applied to a conductive material can be used. As the conductive material constituting the cathode electrode, a carbon-based material containing carbon or graphite as a main component is preferably used from the viewpoint of corrosion resistance and the like.

Sizes of the anode electrode and the cathode electrode are not particularly limited. The sizes of both electrodes may be equal or different. When the microbial fuel cell continues to operate, performance of the cathode electrode decreases. Therefore, it is preferable that an area of the cathode electrode is larger than an area of the anode electrode, and it is more preferable that the area of the cathode electrode is larger than the area of the anode electrode by 1.1 times or more. By increasing the area of the cathode electrode, an operating time of the microbial fuel cell can be increased. In particular, when the microbial fuel cell in the embodiment is a sediment microbial fuel cell installed to purify bottom mud of a river or a lake, the longer the operating time is, the more growth of the strain 60473 can be promoted, which is advantageous for purifying the bottom mud.

The resistor having one end connected to the anode electrode and the other end connected to the cathode electrode is not particularly limited as long as it is a member through which electrons flow from the anode electrode to the cathode electrode due to a potential difference between a chemical reaction caused by a microorganism in which carbon dioxide is generated from an organic substance on the anode electrode side and the electrons are emitted and a reduction reaction occurring on a cathode electrode side, and can be appropriately selected from resistors generally used in cells. The resistor may have a resistance in its entirety from a connection portion with the anode electrode to a connection portion with the cathode electrode, and it is preferable that a conductive member such as a titanium wire having a low internal resistance is used from the connection portion with the anode electrode to the connection portion with the cathode electrode, and an external resistor having a sufficient resistance is provided in the middle. The external resistor is preferably a variable resistor because the external resistor can be adjusted according to a state of the fuel or a state of the microorganism. The resistor and the external resistor are held and fixed on the cathode electrode, and are disposed on the liquid (liquid surface) in an easily visible state.

The microbial fuel cell in the embodiment may be a two-tank microbial fuel cell or a single-tank microbial fuel cell. As structures and members of the two-tank microbial fuel cell and the single-tank microbial fuel cell, structures and members used in known two-tank microbial fuel cells and single-tank microbial fuel cells can be used.

In the case of the two-tank microbial fuel cell, a container, which contains, as a main component, a liquid containing water containing an organic substance, is divided into a tank in which the anode electrode is disposed (anode tank) and a tank in which the cathode electrode is disposed (cathode tank) by a membrane capable of selectively transmitting protons. As the membrane capable of selectively transmitting the protons, a proton exchange membrane (PEM) capable of selectively transmitting $H^+$ can be mainly used. The organic substance and the strain 60473 are contained in the liquid contained in the anode tank.

In the case of the two-tank microbial fuel cell, an amount of oxygen contained in the liquid contained in the anode tank is preferably maintained at about 2% to 10% (v/v) of an amount of oxygen contained in the liquid contained in the cathode tank. In order to maintain an oxygen concentration gradient, the liquid in the cathode tank preferably also contains an oxidant that reacts with protons as a source of oxygen. As the oxidant, potassium ferricyanide, manganese oxide, and the like are used.

In the case of the single-tank microbial fuel cell, the cathode electrode is disposed with one surface in contact with the liquid in the container and the other surface in contact with an atmosphere. Oxygen from the atmosphere is taken in from the surface of the cathode electrode that is in contact with the atmosphere, and a reduction reaction is performed. For example, the cathode electrode can be installed so as to float on the surface of the liquid in the container. A splashed water layer is preferably provided on the side of the cathode electrode in contact with the atmosphere. Examples of the splashed water layer include a coating film of a fluorine-based resin such as polytetrafluoroethylene (PTFE). In addition, it is preferable that a catalyst for the reduction reaction is adhered to the surface of the cathode electrode in contact with the liquid. For example, the surface of the cathode electrode in contact with the liquid can be coated with platinum.

The microbial fuel cell in the embodiment may be a microbial fuel cell that uses organic mud instead of a liquid containing the organic substance. Specifically, the organic mud and the liquid containing water as the main component are contained in the container. In the container, the anode electrode is disposed in the organic mud, and the cathode electrode is disposed within the liquid or on the liquid surface of the liquid. When the cathode electrode is kept floating on the liquid surface of the liquid, a cathode electrode similar to that used in the single-tank microbial fuel cell can be used. On the other hand, when the cathode electrode is disposed within the liquid, it is preferable to contain an oxidant that reacts with the protons as the supply source of oxygen, similarly to the liquid in the cathode tank in the two-tank microbial fuel cell. In the case of the microbial fuel cell using the organic mud, a microorganism group originally contained in the organic mud is also used in the microbial fuel cell. The strain 60473 may be contained in the organic mud, or the strain 60473 may adhere to the anode electrode in advance.

The microbial fuel cell in the embodiment is also preferably a sediment microbial fuel cell used for purifying a water environment. The sediment microbial fuel cell is a microbial fuel cell that generates power by installing the anode electrode in a sediment at the bottom of a river or a lake (bottom mud), and installing the cathode electrode in the water above the sediment. When the sediment microbial fuel cell is operated, an amount of dissolved oxygen and the organic substance in the bottom mud decrease, and it is expected that mud quality of the bottom mud will be improved. Therefore, the sediment microbial fuel cell is installed to purify the bottom mud of the river or the lake. In the case of the sediment microbial fuel cell, the cathode electrode is preferably floated on the liquid surface of the liquid. Although the strain 60473 may be directly injected into the bottom mud in the vicinity of the anode electrode, it is preferable to adhere the strain 60473 to the anode electrode in advance. When the strain 60473 adheres to the anode electrode, the strain 60473 may directly adhere to the anode electrode, or the strain 60473 may be prevented from moving to a position away from the anode electrode.

In the microbial fuel cell in the embodiment, a voltage measurement device is preferably installed in the resistor in order to measure the generated voltage. When an external resistor is used, the voltage measurement device can be installed to sandwich the external resistor. By measuring the generated voltage over time, it is possible to monitor a power generation state, and it is possible to determine a timing to newly supply the strain 60473 or other microorganisms.

The microbial fuel cell in the embodiment is also preferably provided with a supply device for newly supplying the strain 60473 or other microorganisms. When the microbial fuel cell in the embodiment is a sediment microbial fuel cell, it is more important to stably operate for a long period of time, and therefore it is particularly preferable to provide a microbial supply device effective in extending the operating time. The supply device can be constituted by, for example, a supply microorganism container that contains a microorganism solution in which the strain 60473 or other microorganisms are suspended in water, a culture medium, or the like, and a microorganism supply tube that supplies the microorganism solution from the container to the anode electrode.

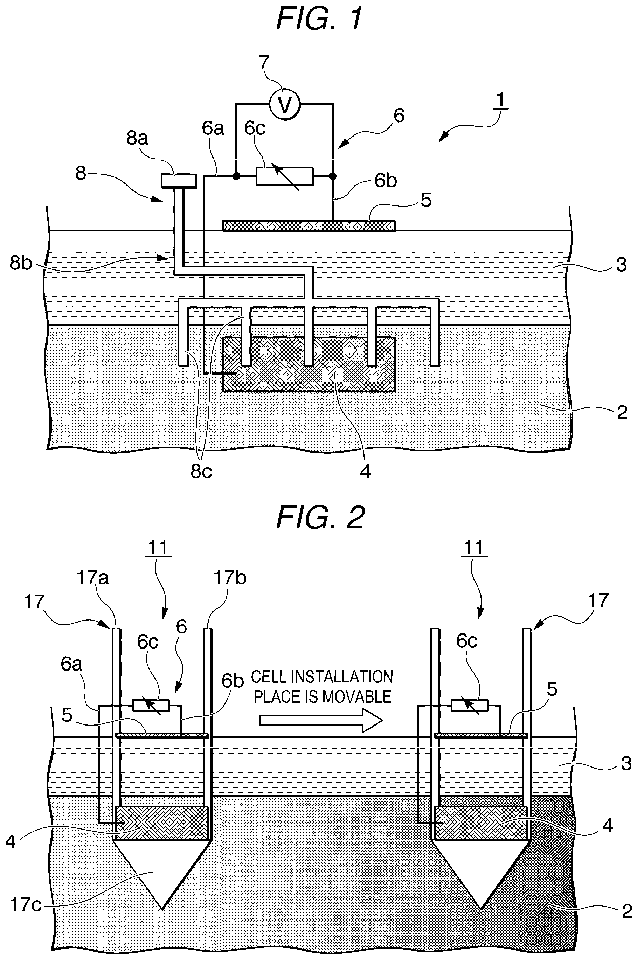

FIG. 1 is a schematic view of one embodiment of the microbial fuel cell in which the microbial supply device is installed.

FIG. 1 shows a microbial fuel cell 1 connected to bottom mud 2 and water 3 of a river or a lake. The microbial fuel cell 1 includes an anode electrode 4, a cathode electrode 5, a resistor 6, a voltage measurement device 7, and a microbial supply device 8.

In the embodiment, the anode electrode 4 is made of graphite felt in which the strain 60473 is planted. The anode electrode 4 is disposed in contact with the bottom mud 2. In the embodiment, the cathode electrode 5 is made of graphite felt supporting a catalyst such as platinum. The cathode electrode 5 is disposed in contact with the water 3. In addition to the graphite felt, a conductor of the electrode may be a porous conductor such as foamed titanium or foamed stainless steel.

The resistor 6 is electrically connected between the anode electrode 4 and the cathode electrode 5. The resistor 6 includes conductive wires 6a and 6b and an external variable resistor 6c. The external variable resistor 6c is connected to the anode electrode 4 via the conductive wire 6a. The external variable resistor 6c is connected to the cathode electrode 5 via the conductive wire 6b. The voltage measurement device 7 that measures the generated voltage is installed across the external variable resistor 6c.

The microbial supply device 8 includes a supply microorganism container 8a and a microorganism supply tube 8b. The supply microorganism container 8a is disposed outside the water 3. The microorganism supply tube 8b extends toward water from the supply microorganism container 8a. A tip of the microorganism supply tube 8b reaches the inside of the bottom mud 2. A portion of the microorganism supply tube 8b extending from the supply microorganism container 8a is a single tube, but a portion on a bottom mud 2 side is branched into a plurality of branch tubes 8c (five in the drawing). The plurality of branch tubes 8c are disposed in a dispersed manner in a planar direction of the anode electrode 4. Open ends of the plurality of branch tubes 8c are disposed around the anode electrode 4 and inside the anode electrode 4. By charging the strain 60473 or a substrate (for example, acetic acid) into the supply microorganism container 8a, the strain 60473 or the substrate can be supplied to the anode electrode 4 as needed through the microorganism supply tube 8b. By providing the plurality of branch tubes 8c, the strain 60473 or the substrate can be supplied in a dispersed manner in the planar direction of the anode electrode 4.

The microbial fuel cell 1 shown in FIG. 1 can supply the strain 60473 or the substrate to the anode electrode 4 as needed by providing the microbial supply device 8. Accordingly, a continuous operation for a long time is possible. Specifically, for example, the transition of the generated voltage is monitored by the voltage measurement device 7 of the microbial fuel cell 1, and when the generated voltage falls below a threshold voltage, the substrate or the strain 60473 is supplied to the anode electrode 4 through the microorganism supply tube 8b of the microbial supply device 8. A threshold value voltage that defines a supply timing is set to, for example, 70% value of a reference voltage that is a generated voltage two weeks after the start of operation of the microbial fuel cell 1. Since it takes about two weeks until a stable operation of the microbial fuel cell 1 is reached, when the voltage decreases by 30% from the generated voltage after the transition to the stable operation, it is determined that a shortage of the substrate or the strain 60473 occurs, and replenishment is performed.

When the microbial fuel cell in the embodiment is a sediment microbial fuel cell, it is preferable that the microbial fuel cell can easily move in bottom mud (sediment). According to the operation of the sediment microbial fuel cell, in a bottom mud portion in which the anode electrode is embedded, the mud quality is improved by a decrease in the amount of organic substances and an increase in the amount of dissolved oxygen, whereas the environment is disadvantageous for the growth of the strain 60473. If the sediment microbial fuel cell can be quickly moved to a place where the mud quality is not improved, the operation can be continued, which is preferable.

FIG. 2 is a schematic view of one embodiment of the microbial fuel cell provided with a support device that facilitates a movement. In FIG. 2, components same as those in FIG. 1 are denoted by the same reference numerals, and a detailed description thereof is omitted.

A sediment microbial fuel cell 11 shown in FIG. 2 includes the anode electrode 4, the cathode electrode 5, the resistor 6, and a support device 17.

The support device 17 includes two support bodies 17a and 17b and an installation fixing portion 17c fixed to lower ends of the support bodies 17a and 17b. Each of the support bodies 17a and 17b is a rod-shaped or plate-shaped body extending along a vertical direction. The installation fixing portion 17c is, for example, a conical structure disposed such that a tip thereof faces a lower side in a depth direction (a lower side in the drawing). An angle of the conical tip of the installation fixing portion 17c is preferably an acute angle. The installation fixing portion 17c may have a shape having a portion protruding downward in the depth direction so that the support device 17 can be easily embedded in the bottom mud 2. The installation fixing portion 17c may have, for example, a pyramid shape. By inserting the installation fixing portion 17c toward the bottom mud 2, the sediment microbial fuel cell 11 can be easily inserted and fixed to the bottom mud 2.

The anode electrode 4 and the cathode electrode 5 are connected to the two support bodies 17a and 17b. In the example shown in FIG. 2, the anode electrode 4 and the cathode electrode 5 are disposed between the support bodies 17a and 17b disposed at an interval in a horizontal direction. The support bodies 17a and 17b are connected to both ends of the anode electrode 4 in the horizontal direction and both ends of the cathode electrode 5 in the horizontal direction, respectively. That is, the anode electrode 4 and the cathode electrode 5 are fixed between the two support bodies 17a and 17b in such a manner that the anode electrode 4 is connected to the upper portion and the cathode electrode 5 is connected to the lower portion in parallel.

The support device 17 may be implemented to include only the support body 17a, and in this case, one end of the anode electrode 4 and one end of the cathode electrode 5 are connected to the support body 17a.

The support device 17 includes a position adjustment mechanism capable of adjusting and fixing positions and intervals of the anode electrode 4 and the cathode electrode 5 supported in parallel by the support bodies 17a and 17b. Accordingly, relative positions of the anode electrode 4, the cathode electrode 5, and the installation fixing portion 17c can be adjusted according to a water depth (depth), an installation position in the bottom mud 2, and the like.

The sediment microbial fuel cell 11 shown in FIG. 2 has a configuration in which both the anode electrode 4 and the cathode electrode 5 are supported by a single support device 17, and thus transportation and installation can be performed efficiently. When the sediment microbial fuel cell 11 is operated, an amount of dissolved oxygen and the organic substance in the bottom mud 2 decrease, and it is expected that mud quality of the bottom mud 2 will be improved. However, when the state of the bottom mud 2 is improved, the amount of oxygen and the organic substance of the bottom mud 2 are reduced, and therefore, the environment becomes disadvantageous for the growth of the facultatively anaerobic strain 60473. When the cell can be quickly moved to a place where the mud quality is not improved (a place where the amount of dissolved oxygen and the amount of organic substance are large), an efficient operation can continue. In the sediment microbial fuel cell 11 in the embodiment, since the anode electrode 4 and the cathode electrode 5 can be integrally moved and the support device 17 having an acute tip is provided, the sediment microbial fuel cell 11 can be easily moved to an environment where the strain 60473 is easily grown.

Microbial Electrolysis Cell Composition

A microbial electrolysis cell composition in the embodiment contains the strain 60473 and an organic substance, and is a composition that is used as an organic substance serving as a fuel for a microbial electrolysis cell or is added to the fuel. The microbial electrolysis cell composition can also be used as the bioaugmentation microbial preparation. A form of the microbial electrolysis cell composition in the embodiment is not particularly limited, and may be a solid substance such as a granule or a lump, a paste, a gel, or a suspension.

The organic substance contained in the microbial electrolysis cell composition in the embodiment is not particularly limited as long as it serves as the fuel for the microbial electrolysis cell, and the same organic substances as listed as the organic substance contained in the microbial fuel cell composition can be used.

The microbial electrolysis cell composition in the embodiment may contain one or more types of microorganisms other than the strain 60473. The microorganisms may be electrochemically active bacteria or may be other than the electrochemically active bacteria. Any microorganism can be used as long as it can grow in an environment where the strain 60473 can grow, and is preferably an anaerobic bacterium, and may also be a microaerobic bacterium. Examples of the microorganisms other than the strain 60473 contained in the microbial electrolysis cell composition include electrochemically active bacteria and anaerobic acetic acid-producing bacteria.

Microbial Electrolysis Cell

The microbial electrolysis cell in the embodiment is characterized in that the strain 60473 is used for decomposition of an organic substance as a fuel. In the microbial electrolysis cell according to the embodiment, the microorganism used for the decomposition of the organic substance may be only the strain 60473, or the strain 60473 may be used in combination with other microorganisms. By using the strain 60473 and the anaerobic acetic acid-producing bacteria in combination, acetic acid essential for power generation and hydrogen production by the strain 60473 is stably supplied, and the power generation and the hydrogen production by the strain 60473 can be stably performed for a longer period of time. By using a wide variety of microorganisms capable of decomposing the organic substance in combination, the organic substance serving as the fuel can be efficiently decomposed into a state that can be easily used by the strain 60473. It is also preferable to use the electrochemically active bacteria other than the strain 60473 in combination because it can enhance hydrogen production efficiency for the organic substance serving as the fuel.

In the microbial electrolysis cell in the embodiment, an amount of the strain 60473 to be used is not particularly limited, and is appropriately adjusted in consideration of a type and a concentration of the organic substance used as the fuel, a type of anode electrode, presence or absence of adhesion to the anode electrode, and the like. When the strain 60473 is dispersed in a liquid containing the organic substance, the amount of the strain 60473 per gram of the liquid can be, for example, 10,000 cells/g to 100,000,000 cells/g, and is preferably 100,000 cells/g to 10,000,000 cells/g. When the strain 60473 adheres to the anode electrode, the amount of the strain 60473 per 1 cm$^2$ of a surface area of the anode electrode can be, for example, 10,000 cells/g to 100,000,000 cells/g, and is preferably 100,000 cells/g to 10,000,000 cells/g.

In the microbial electrolysis cell in the embodiment, when using the strain 60473 and other electrochemically active bacteria in combination, a ratio of the strain 60473 to a total amount of the electrochemically active bacteria in the microbial electrolysis cell is not particularly limited, and is preferably 1 mass% or more, more preferably 10 mass% or more, and even more preferably 30 mass% or more. Since the strain 60473 has high electrolytic capacity, even when a small amount is added to a microbial electrolysis cell using the electrochemically active bacteria in the related art, an effect of increasing a hydrogen production amount can be expected.

The electrolytic capacity of the strain 60473 decreases as a cell usage time increases. Therefore, in the microbial electrolysis cell according to the embodiment, it is preferable to newly supply the strain 60473 at a timing when the electrolytic capacity of the strain 60473 decreases after the start of the operation of the cell. In addition to the supply of the strain 60473, anaerobic acetic acid-producing bacteria and other microorganisms can also be supplied.

The timing to newly supply the strain 60473 can be determined, for example, by monitoring a transition of a generated voltage by the microbial electrolysis cell. In the microbial electrolysis cell, the generated voltage becomes stable after a while after the start of operation. Based on a generated voltage value in a state in which the generated voltage is stable (stable generated voltage value), when the generated voltage value falls below a preset threshold value (for example, a 70% value of the stable generated voltage value), the strain 60473 is newly supplied, so that the power generation and the hydrogen production can be stably performed for a long period of time. The new strain 60473 may be supplied every predetermined time from the start of the operation of the cell.

The microbial electrolysis cell in the embodiment uses the strain 60473, and only needs to include an anode electrode, a cathode electrode, a potential control device connected to both electrodes, through which electrons flow, and an organic substance serving as a fuel, which are essential components of the cell, and other structures are not particularly limited. Specifically, the microbial electrolysis cell according to the embodiment includes a liquid containing water containing an organic substance as a main component, a container that contains the liquid, a conductive anode electrode disposed in the liquid, a conductive cathode electrode disposed with at least one surface thereof in contact with the liquid, and a potential control device having one end connected to the anode electrode and the other end connected to the cathode electrode, through which electrons flow. Microorganisms including the strain 60473 are contained in the liquid in which the anode electrode is disposed. Examples of the organic substance serving as the fuel include the organic substances same as those contained in the microbial electrolysis cell composition. In the microbial electrolysis cell in the embodiment, it is preferable to use an organic substance containing phosphorus, nitrogen, and sulfide.

On an anode electrode side, the organic substance serving as the fuel is decomposed by the microorganisms to generate carbon dioxide, and during the reaction, the electrochemically active bacteria such as the strain 60473 emit electrons. The anode electrode is not particularly limited as long as it is an electrode made of a conductive material capable of receiving electrons produced from electrochemically active bacteria and delivering the electrons to the cathode electrode through the potential control device. In the microbial electrolysis cell according to the embodiment, a conductive carbon-based anode electrode containing carbon or graphite as a main component is preferable from the viewpoint of good adhesion of microorganisms, and an anode electrode made of a carbon cloth (a cloth made by spinning carbonized fibers obtained by firing petroleum or acrylic fibers) is more preferable from the viewpoint of a large surface area. In addition, in the microbial electrolysis cell according to the embodiment, it is also preferable that the anode electrode is made of a conductive metal material with a lower internal electrical resistance than the cathode electrode. Specifically, in the microbial electrolysis cell in the embodiment, an anode electrode made of metal such as stainless steel, iron, or magnesium, and having the strain 60473 adhered to the surface is also preferable.

In the cathode electrode, a reduction reaction is caused by the electrons received from the anode electrode through the potential control device and protons diffused from the anode electrode, and hydrogen is produced. As the cathode electrode, an electrode in which a catalyst for a reduction reaction such as platinum is applied to a conductive material can be used. As the conductive material constituting the cathode electrode, a carbon-based material containing carbon or graphite as a main component is preferably used from the viewpoint of corrosion resistance and the like.

Sizes of the anode electrode and the cathode electrode are not particularly limited. The sizes of both electrodes may be equal or different. When the microbial electrolysis cell continues to operate, performance of the cathode electrode decreases. Therefore, it is preferable that an area of the cathode electrode is larger than an area of the anode electrode, and it is more preferable that the area of the cathode electrode is larger than the area of the anode electrode by 1.1 times or more. By increasing the area of the cathode electrode, an operating time of the microbial electrolysis cell can be increased.

The potential control device having one end connected to the anode electrode and the other end connected to the cathode electrode is not particularly limited as long as it is a member through which electrons flow from the anode electrode to the cathode electrode due to a potential difference between a chemical reaction caused by a microorganism in which carbon dioxide is generated from an organic substance on the anode electrode side and the electrons are emitted and a reduction reaction occurring on a cathode electrode side, and can be appropriately selected from potential control devices generally used in the microbial electrolysis cell. Examples of the potential control device include a potentiostat that uses a working electrode (anode electrode), a counter electrode (cathode electrode), and a reference electrode. The potential control device may have a resistance in its entirety from a connection portion with the anode electrode to a connection portion with the cathode electrode, and it is preferable that a conductive member such as a titanium wire having a low internal resistance is used from the connection portion with the anode electrode to the connection portion with the cathode electrode, and an external resistor having a sufficient resistance is provided in the middle. The external resistor is preferably a variable resistor because the external resistor can be adjusted according to a state of the fuel or a state of the microorganism. The potential control device and the external resistor are held and fixed on the cathode electrode, and are disposed on the liquid (liquid surface) in an easily visible state.

The microbial electrolysis cell in the embodiment may be a two-tank microbial electrolysis cell or a single-tank microbial electrolysis cell. As structures and members of the two-tank microbial electrolysis cell and the single-tank microbial electrolysis cell, structures and members used in known two-tank microbial electrolysis cells and single-tank microbial electrolysis cells can be used.

In the case of the two-tank microbial electrolysis cell, a container, which contains, as a main component, a liquid containing water containing an organic substance, is divided into a tank in which the anode electrode is disposed (anode tank) and a tank in which the cathode electrode is disposed (cathode tank) by a membrane capable of selectively transmitting protons. As the membrane capable of selectively transmitting the protons, a proton exchange membrane (PEM) capable of selectively transmitting H+ can be mainly used. The organic substance and the strain 60473 are contained in the liquid contained in the anode tank.

In the case of the two-tank microbial electrolysis cell, an amount of oxygen contained in the liquid contained in the anode tank is preferably maintained at about 2% to 10% (v/v) of an amount of oxygen contained in the liquid contained in the cathode tank. In order to maintain an oxygen concentration gradient, the liquid in the cathode tank preferably also contains an oxidant that reacts with protons as a source of oxygen. As the oxidant, potassium ferricyanide, manganese oxide, and the like are used.

The microbial electrolysis cell in the embodiment may be a microbial electrolysis cell that uses organic mud instead of a liquid containing the organic substance. Specifically, the organic mud and the liquid containing water as the main component are contained in the container. In the container, the anode electrode is disposed in the organic mud, and the cathode electrode is disposed within the liquid. As the cathode electrode, it is preferable to contain an oxidant that reacts with the protons as the supply source of oxygen, similarly to the liquid in the cathode tank in the two-tank microbial electrolysis cell. In the case of the microbial electrolysis cell using the organic mud, a microorganism group originally contained in the organic mud is also used in the microbial electrolysis cell. The strain 60473 may be contained in the organic mud, or the strain 60473 may adhere to the anode electrode in advance.

The microbial electrolysis cell in the embodiment is also preferably a sediment microbial electrolysis cell used for purifying a water environment. The sediment microbial electrolysis cell is a microbial electrolysis cell that generates power and produces hydrogen by installing the anode electrode in a sediment at the bottom of a river or lake (bottom mud), and installing the cathode electrode in the water above the sediment. When the sediment microbial electrolysis cell is operated, an amount of dissolved oxygen and the organic substance in the bottom mud decrease, and it is expected that mud quality of the bottom mud will be improved. Therefore, the sediment microbial electrolysis cell can be installed in the river or the lake in the same manner as the sediment microbial fuel cell described above to purify the bottom mud.

In the microbial electrolysis cell in the embodiment, a voltage measurement device is preferably installed in the potential control device in order to measure the generated voltage. When an external resistor is used, the voltage measurement device can be installed to sandwich the external resistor. By measuring the generated voltage over time, it is possible to monitor a power generation state, and it is possible to determine a timing to newly supply the strain 60473 or other microorganisms.

The microbial electrolysis cell in the embodiment is also preferably provided with a supply device for newly supplying the strain 60473 or other microorganisms. When the microbial electrolysis cell in the embodiment is a sediment microbial electrolysis cell, it is more important to stably operate for a long period of time, and therefore it is particularly preferable to provide a microbial supply device effective in extending the operating time. The supply device can be constituted by, for example, a supply microorganism container that contains a microorganism solution in which the strain 60473 or other microorganisms are suspended in water, a culture medium, or the like, and a microorganism supply tube that supplies the microorganism solution from the container to the anode electrode.

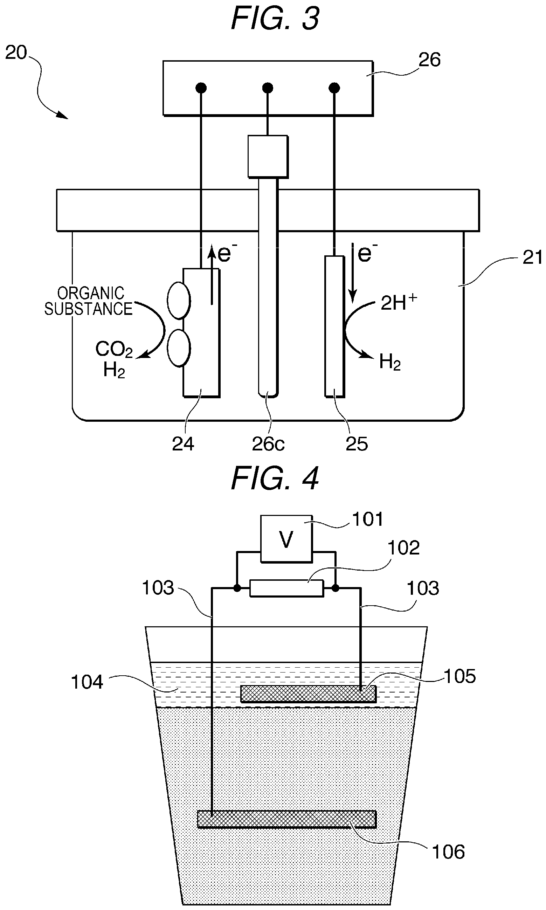

FIG. 3 is a schematic view of one embodiment of a three-electrode single-tank microbial electrolysis cell. A microbial electrolysis cell 20 includes an anode electrode 24, a cathode electrode 25, and a potentiostat 26 in a container 21. The potentiostat 26 has a reference electrode 26c. In the anode electrode 24, an organic substance is decomposed by a microorganism such as the strain 60473, and generated electrons move to the cathode electrode 25 through the potentiostat 26. In the cathode electrode 25, electrons react with protons to generate hydrogen.

Examples

Next, the present disclosure will be described in more detail with reference to Examples and Reference Examples, and the present disclosure is not limited to the following Examples and the like.

Example 1

An electrochemically active bacterium having power generation capacity that can be used in a microbial fuel cell was isolated and identified from mud on the shores of Lake Suwa (Okaya City, Nagano Prefecture).

(1) Isolation of Strain 60473

Specifically, first, a mud cell [101: voltage measurement data logger, 102: external resistor, 103: titanium wire, 104: water, 105: cathode (platinum-coated graphite felt), 106: anode (graphite felt), 107: mud] shown in FIG. 4 was prepared. The mud cell was operated in an incubator at 30°C while measuring a voltage across an external resistance (10 k$\Omega$) to grow power generating bacteria on the anode. After the voltage was increased to 0.5 V or more, a part of the anode was cut off, and the cut anode was placed in a liquid medium of a DSM826 medium (manufactured by DSMZ) and stirred, thereby suspending an adhesive material. A diluted solution of the obtained suspension was applied to an agar plate of the DSM826 medium, placed in an AneroPack Pouch (manufactured by Mitsubishi Gas Chemical Co., Ltd.), and colonies were formed at 30°C. Red colonies formed on the plate were inoculated into a DSM826 liquid medium in an anaerobic vial in which a gas phase was replaced with nitrogen gas (99.999% (v/v)) and cultured. The culture was cultured again on the agar plate of the DSM826 medium to form colonies, and the formed colonies were cultured again on the DSM826 liquid medium. The operation was repeated until only colonies with the same shape were formed on the plate. The anaerobic bacteria colonies formed on the plate were named the strain 60473.

(2) Evaluation of Current Generating Ability of Strain 60473

The strain 60473 was cultured in the DSM826 liquid medium to obtain the culture. The culture was placed in a three-electrode single-tank electrochemical cell, and the generated current was measured. In the electrochemical cell, the working electrode is graphite felt (2.00 $cm^2$, manufactured by Nippon Carbon Co., Ltd.), the reference electrode is an Ag/AgCl electrode (RE-T8A, manufactured by EC Frontier), the counter electrode is a platinum wire (length of 12 cm, thickness of 0.3 mm, manufactured by Nilaco Corporation), and an electrolyte is a fumaric acid-free DSM826 liquid medium (65 mL). A culture was added to the electrolyte so that turbidity at 600 nm was 0.02, the cell was connected to a potentiostat (VMP3, manufactured by Biologic), a working electrode potential was set to -0.2 V vs. Ag/AgCl electrode, and current measurement was started. As a comparison, a current generated by the strain PCA, which is known as a highly capable power generating bacterium, was measured (n = 2).

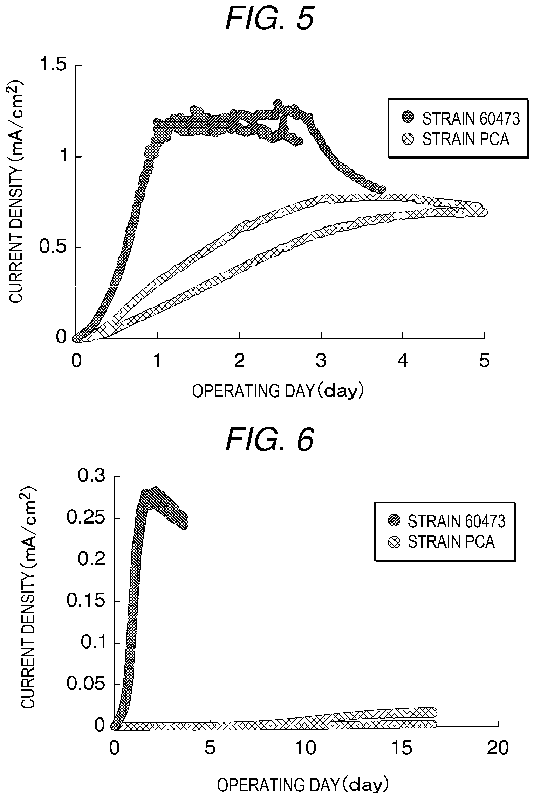

FIG. 5 shows current measurement results in the electrochemical cell planted with the strain 60473 and the electrochemical cell planted with the strain PCA. As shown in FIG. 5, a maximum value of the current generated by the strain 60473 was about twice as high as a maximum value of the strain PCA.

The working electrode of the electrochemical cell was replaced with glassy carbon (1 $cm^2$, manufactured by BAS), and the current was measured in the same manner as above (n = 2). Results are shown in FIG. 6. As a result, a current value is increased only to about 0.02 mA/$cm^2$ for the strain PCA, but is increased to about 0.28 mA/$cm^2$ for the strain 60473. These results indicate that the strain 60473 is able to efficiently interact electrochemically with the smooth glassy carbon electrode. At this time, when the bacteria adhered to an electrode surface were stained with a fluorescent dye (4,6-diamidino-2-phenylindole, DAPI; 0.1 $\mu$g/mL) and observed using a confocal microscope (FV1200/BX61WI system, manufactured by Olympus Corporation), it is confirmed that many cells in the strain 60473 are adhered to the smooth electrode. An amount of bacteria adhered to the glassy carbon electrode is compared based on a DAPI staining intensity.

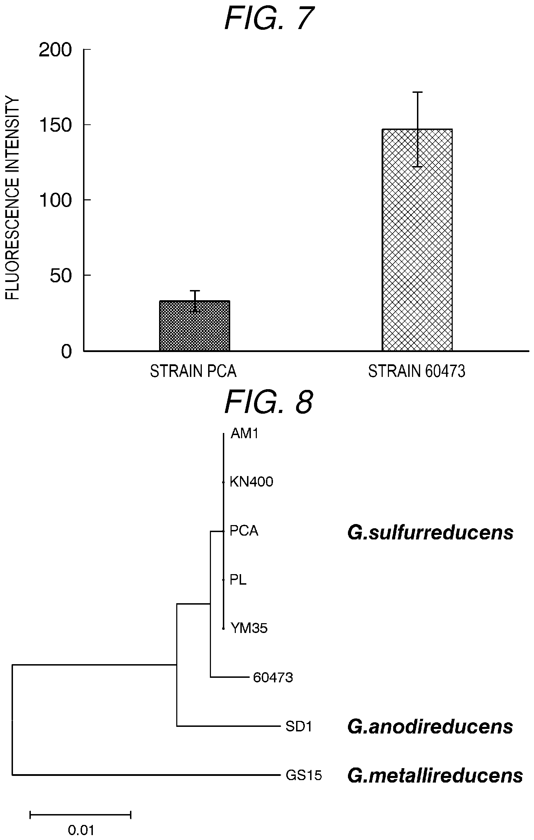

Results are shown in FIG. 7. The number of the strain 60473 adhered to the glassy carbon electrode is nearly three times as large as that of the strain PCA.

(3) Genome Analysis of Strain 60473

A genome sequence was determined in order to identify the species of strain 60473 from the genome sequence and further clarify its genomic characteristics. The strain 60473 was cultured in the DSM826 medium, and genomic DNA was extracted from 100 mL of the culture according to a method of Fujikawa et al. (Fujikawa et al., FEMS Microbiology Letters, 2021, vol. 368(17), fnab119.). It is confirmed by agarose gel electrophoresis that high-molecular genomic DNA can be extracted, and the entire genome sequence is determined using a Sequel Ile system (manufactured by PacBio). Table 1 shows genome nucleotide sequence analysis results obtained by comparing the strain 60473 with the strain PCA.

Table 1

|  | Strain 60473 | Strain PCA |
| --- | --- | --- |
| Genome size (bp) | 3793644 | 3814128 |
| CDS | 3429 | 3711 |
| G+C content (%) | 61.1 | 60.5 |
| rRNA operon | 2 | 2 |
| Plasmid | 0 | 0 |

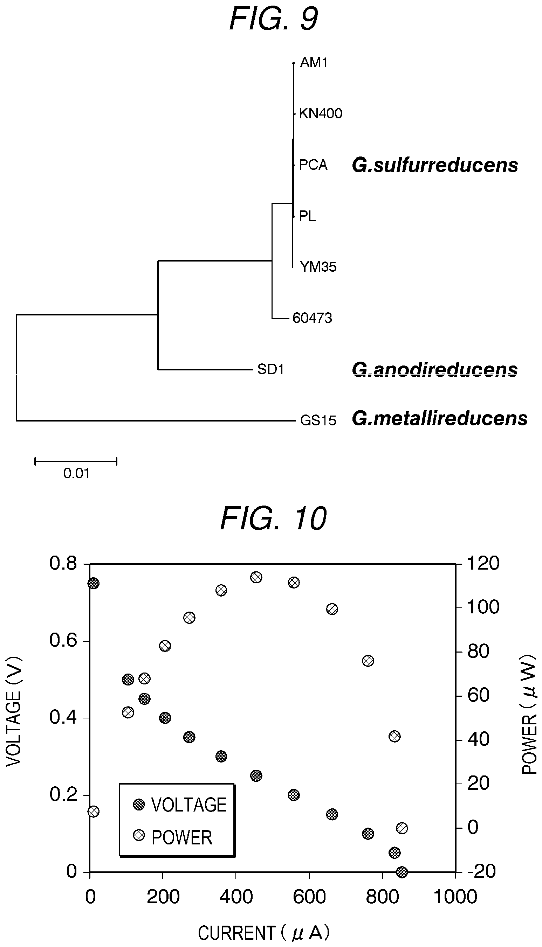

In order to clarify molecular phylogenetic characteristics of the strain 60473, BLAST analysis in an NCBI database was performed using a 16S rRNA gene in the genome. As a result, the closest relative of the strain 60473 was the strain PCA, and homology (sequence identity) with the strain was 99.48%. Therefore, in order to clarify a phylogenetic relationship with the known Geobacter sulfluducens, a phylogenetic tree was created using the 16S rRNA gene and a gyrB gene (FIGS. 8 and 9). As a result, although the strain 60473 belongs to the genus Geobacter, further investigation is considered necessary to determine whether it belongs to Geobacter sulfluducens.

Example 2

The mud cell is an experimental device that mimics a sediment microbial fuel cell (sMFC), which is a power generation device in an environment. The sMFC is a device that generates power using an organic substance in the sediment (mud and bottom mud) as a fuel, and can reduce the organic substance in the sediment by generating power. Therefore, in order to investigate whether the strain 60473 can be used to improve performance of the sMFC, a mud cell is created using bottom mud from Lake Suwa, and an attempt is made to introduce the strain 60473.

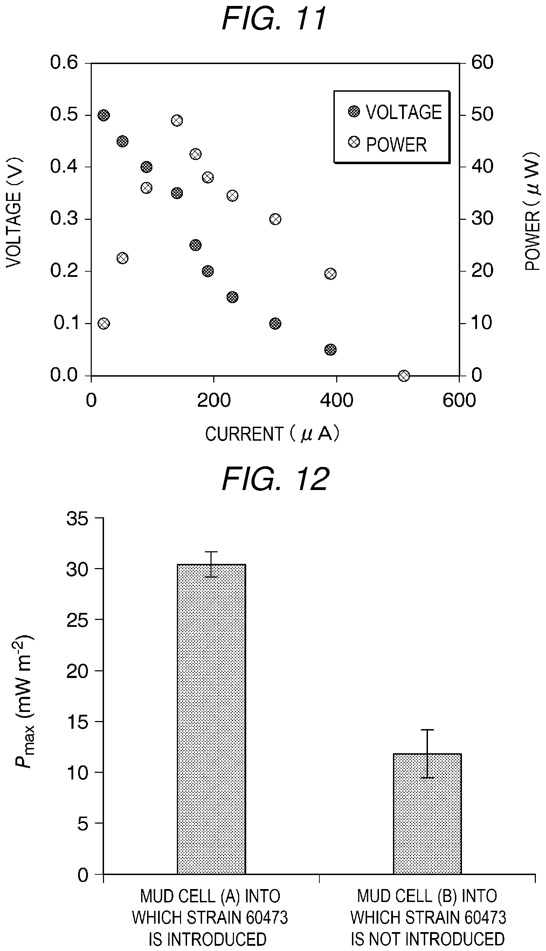

The mud cell used has a structure same as that schematically shown in FIG. 4. The mud cell used is made of mud (approximately 400 mL), an anode (graphite felt, area 36 cm$^2$), and a cathode (platinum-coated graphite felt, area 24 cm$^2$), and was operated with an external resistance of 10 k$\Omega$ connected. One day after the start of operation, a bacterial solution concentrated by centrifuging 250 mL of a culture solution of the strain 60473 cultured in the DSM826 liquid medium was injected around the anode, and the operation was continued. Results of polarization analysis conducted on the fifth day after the start of operation are shown in FIGS. 10 and 11. FIG. 10 shows polarization analysis results (n = 2) of the mud cell into which the strain 60473 is introduced. FIG. 11 shows polarization analysis results (n = 2) of the mud cell into which the strain 60473 is not introduced. A maximum power density ($P_{max}$ mW/m$^2$) is calculated based on the polarization analysis results shown in FIGS. 10 and 11, and a comparison thereof is shown in FIG. 12. In the drawings, an error line indicates a standard deviation. By introducing the strain 60473, the maximum power density of the mud cell is dramatically improved. These results indicate that cell performance can be further improved by performing bioaugmentation using the strain 60473 on an existing microbial fuel cell, particularly, on the sMFC.

Example 3

The power generation capacity of the strain 60473 was compared with known Geobacter bacteria. For comparison, the strain PCA used in Example 1, a Geobacter sulfurreducens strain KN400 (KEGG GENOME: Geobacter sulfurreducens KN400), and a Geobacter sulfurreducens strain YM18 described in Ochiai et al., Bioresource Technology, 2023, vol. 386, 129508 were used.

(1) Evaluation of Current Generating Ability

Specifically, in the same manner as in "(2) Evaluation of current generating ability of strain 60473" in Example 1, the Geobacter bacteria to be evaluated were planted in a three-electrode single-tank electrochemical cell, and the generated current was measured (n = 2).

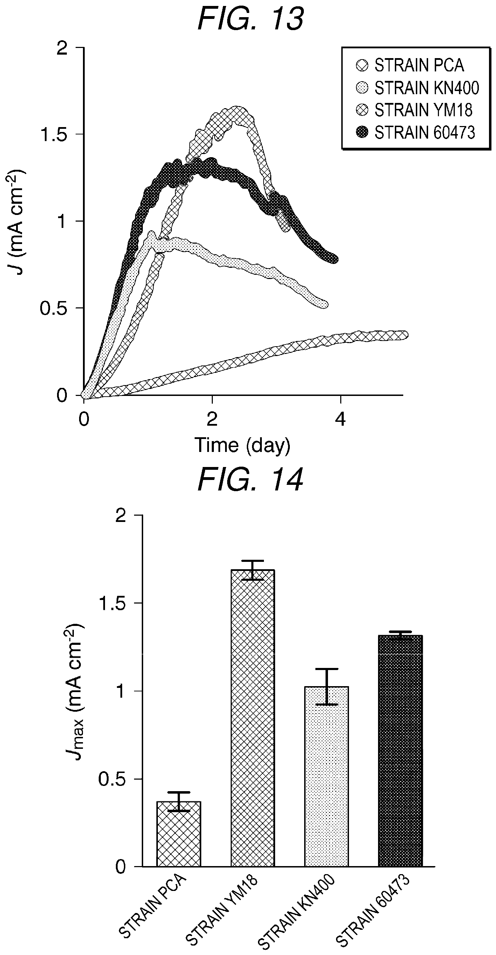

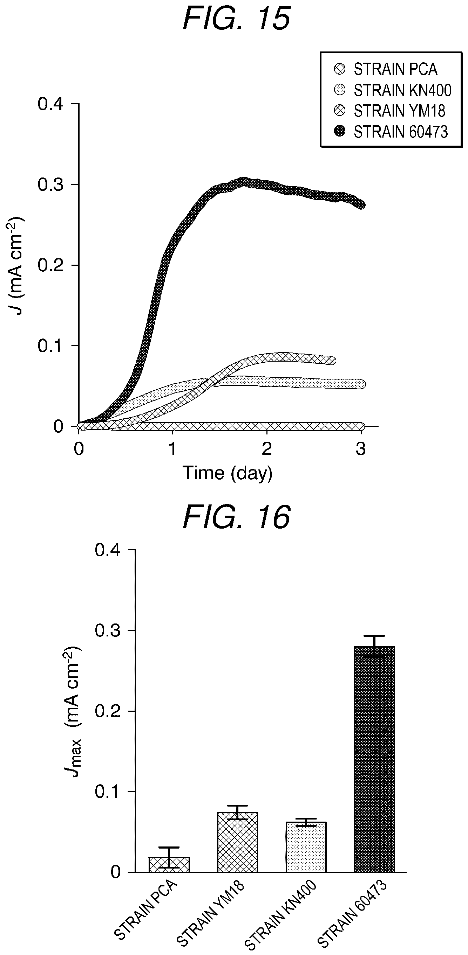

Results of measuring the current density over time when graphite felt is used as the working electrode (anode electrode) are shown in FIG. 13, and a maximum current density is shown in FIG. 14, respectively. Results of measuring the current density over time when glassy carbon is used as the working electrode (anode electrode) are shown in FIG. 15, and a maximum current density is shown in FIG. 16, respectively. As shown in FIGS. 13 and 14, when a graphite felt electrode is used, the strain 60473, the strain KN400, and the strain YM18 all show a relatively higher current density than the strain PCA, and among these, the strain YM18 has the highest current density. In the electrochemical cell inoculated with the strain 60473 and the strain KN400, it is shown that the current density increases relatively rapidly compared to the strain YM18. On the other hand, as shown in FIGS. 15 and 16, when a glassy carbon electrode is used, the strain 60473 generates the highest current among these strains, and the maximum current density ($J_{max}$) of the strain 60473 is more than three times that of the strain KN400 and the strain YM18.

In the same manner as in Example 1, the graphite felt electrode and glassy carbon electrode after the current generation experiment were subjected to the DAPI staining and observed using the confocal microscope. As a result, the strain KN400 and the strain YM18 formed thick biofilms on graphite fibers of the graphite felt electrode. Although the biofilm of the strain 60473 on a graphite felt fiber was thinner than that of the strain KN400 and the strain YM18, a cell density of the biofilm of the strain 60473 was the highest. Further, the strain 60473 also formed a high-density biofilm on the glassy carbon electrode, and the density was much higher than the biofilms of other strains. Meanwhile, the strain KN400 formed a biofilm with low cell density that was evenly distributed on the glassy carbon electrode, and the biofilm of the strain YM18 only partially covered a surface of the glassy carbon electrode. From these observations, it is concluded that the high current density of the strain 60473 is related to its ability to form a high-density biofilm on the graphite surface.

(2) Physiological Traits of Geobacter Bacteria

For the strain 60473, the strain KN400, the strain YM18, and the strain PCA, among the physiological traits thought to be related to biofilm formation and current generation, graphite affinity (GAI), cell hydrophobicity (HPI), and cytochrome content per protein (CCI) were examined.

Measurement of GAI (%)

The GAI (%) of each Geobacter bacteria was measured by the following method.

First, a test microorganism was grown by anaerobic culture in the DSM826 medium, and then washed with a DSM826 buffer (a DSM826 medium containing no acetic acid salt, fumaric acid salt and mineral solution). Next, the test microorganism was suspended in 3.5 mL of the DSM826 buffer so that an optical density at a wavelength of 600 nm (optical density 600 nm: $OD_{600}$) was 0.3 to prepare a fungus body suspension. The $OD_{600}$ was measured with a digital colorimeter (miniphoto 518R, manufactured by Taitec Corporation).

Graphite felt (0.5 cm$^2$ × 5 mm) was added to the prepared fungus body suspension, and a mixture thereof was stirred at 30°C and 200 rpm. The $OD_{600}$ was measured using the digital colorimeter 60 minutes after the start of stirring.

The GAI (%) was calculated based on an obtained measured value and the following formula. In the formula, $A_1$ is the $OD_{600}$ of the fungus body suspension after stirring, and Ao is the $OD_{600}$ (0.3) of the fungus body suspension before stirring.

$$[GAI\ (\%)] = (1 - A_1/A_0) \times 100$$

Measurement of HPI (%)

The HPI (%) of each Geobacter bacteria was measured by the following method.

The test microorganism was suspended in 4.8 mL of a sodium chloride aqueous solution (0.15 M) so that the optical density at the wavelength of 600 nm (optical density 600 nm: $OD_{600}$) was 0.3 to prepare a fungus body suspension. The $OD_{600}$ was measured with a digital colorimeter (miniphoto 518R, manufactured by Taitec Corporation).

Hexadecane (0.8 mL) was added to the prepared fungus body suspension, and a mixture thereof was stirred for 60 seconds using a vortex mixer. Thereafter, the fungus body suspension was left overnight to completely separate two phases, and then an upper layer was removed by decantation, and the $OD_{600}$ in a lower layer was measured.

The HPI (%) was calculated based on a ratio of cells that transitioned to a hexadecane phase and based on the following formula. In the formula, $A_2$ is the $OD_{600}$ of the fungus body suspension in an aqueous phase after mixing the fungus body suspension with hexadecane, and Ao is the $OD_{600}$ (0.3) of the fungus body suspension before stirring.

$$[HPI (\%)] = (1 - A_2/A_0) \times 100$$

Measurement of CCI (/mg)

The CCI (/mg) of each Geobacter bacteria was measured by the following method.

First, a test microorganism was grown by anaerobic culture in the DSM826 medium, and then centrifuged (10,000 × g, 15 minutes) to collect a microorganism pellet. The obtained microorganism pellet was washed with a TE buffer and then suspended in the TE buffer containing 1 mg/mL lysozyme. The obtained fungus body suspension was homogenized using a homogenizer, and then incubated at 37°C for 1.5 hours, further added with Triton X-100 (1% w/v), and incubated at a room temperature for 10 minutes.

An absorption spectrum of a supernatant of the fungus body suspension after incubation was measured in a range of 700 nm to 350 nm using a spectrophotometer (UH5300, manufactured by Hitachi, Ltd).

Next, in order to measure the absorption spectrum of reduced cytochrome, sodium bisulfite was added to the fungus body suspension so that a final concentration was 68 mM, and an absorbance of a Soret band peak (about 420 nm) of reduced cytochrome was measured.

Further, a protein content ($\mu$g) of the fungus body suspension was measured using a commercially available BCA assay kit (Micro BCA protein assay kit, manufactured by Thermo Fisher Scientific).

The CCI (/mg) was calculated based on an obtained measured value and the following formula. In the formula, $A_1$ is the $OD_{600}$ of the fungus body suspension after stirring, and Ao is the $OD_{600}$ (0.3) of the fungus body suspension before stirring. In the formula, $A_{Soret}$ is the absorbance of the Soret band peak of reduced cytochrome in the fungus body suspension, and PC is the protein content ($\mu$g) of the fungus body suspension.

$$[CCI (/mg)] = A_{Soret}/PC$$

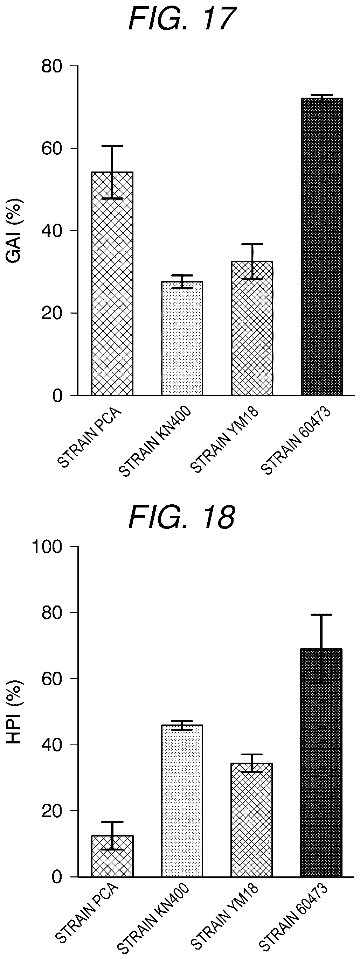

Measurement results of the GAI, the HPI, and the CCI of each Geobacter bacteria are shown in FIGS. 17 to 19. The CCI is higher for the strain YM18 and the strain 60473 than for the strain PCA and the strain KN400. That is, the strain 60473 can generate relatively a high current density with both the graphite felt electrode and the glassy carbon electrode, whereas the strain YM18 generates a high current density only with the graphite felt electrode. The reason why the strain YM18 can form an electrochemically active biofilm (EABF) with the graphite felt electrode is that cells were physically trapped inside graphite felt fiber, and it is considered that the strain does not easily adhere to a flat surface. On the other hand, it is interesting that the strain 60473 forms a high-density biofilm on a flat and slippery surface of the glassy carbon electrode, and high GAI and high HPI characteristics of the strain 60473 promote the action.

Example 4

In order to evaluate usefulness of the strain 60473 in bioaugmentation of the microbial electrolysis cell, the cell was operated using starch as a main anode substrate, and a power generation amount and a hydrogen production amount were examined.

Specifically, soil was inoculated into four single-layer electrolysis cells, the cells were operated using starch as the main anode substrate (MEC1 to MEC4), the strain 60473 was ingested into two electrolysis cells (MEC3 and MEC4) one day after the start of the operation, and the operation was continued. The remaining two electrolysis cells (MEC1 and MEC2) were not ingested with the strain 60473 and continued to operate as controls without bioaugmentation. During the operation, the electrolysis cell was intermittently replaced with an electrolyte to add the substrate (starch). A time from the time of electrolyte solution replacement to the next time of electrolyte solution replacement was defined as "one cycle", and the operation was continued until a sixth cycle (after performing the electrolyte solution replacement five times).

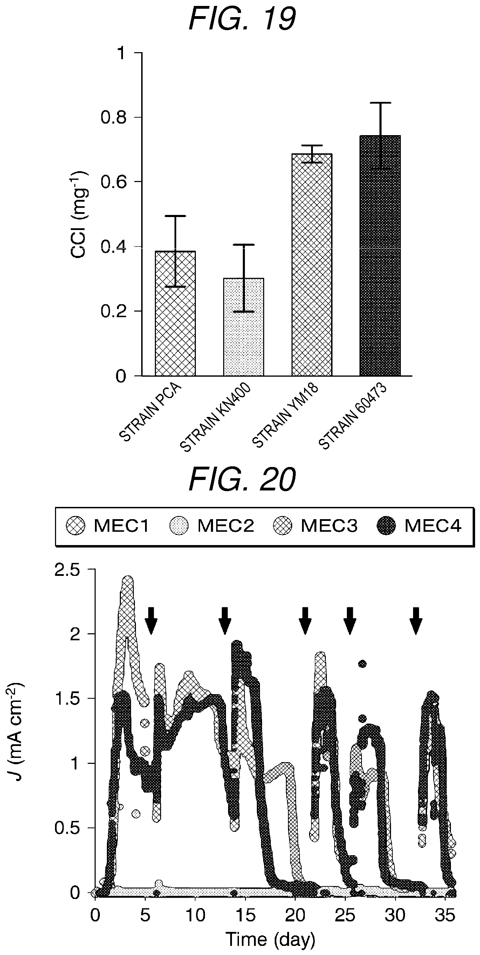

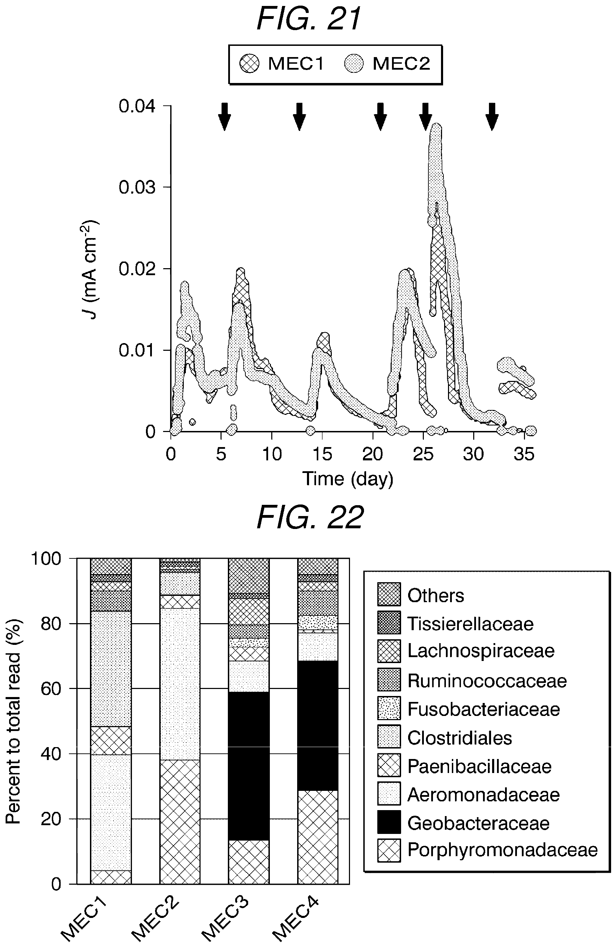

Results obtained by measuring the power generation amount (current density) of each cell over time are shown in FIGS. 20 and 21. FIG. 21 is an enlarged view of a vertical axis of measurement data in FIG. 20. In FIGS. 20 and 21, time points indicated by arrowheads indicate time points when the electrolyte is replaced.

After the operation was completed, the biofilm formed on a surface of the working electrode (anode electrode) of each cell was collected, constituent bacteria were classified by genus, and a composition was examined. An amount of each bacteria was measured by a quantitative PCR method as in Ochiai et al., Bioresource Technology, 2023, vol. 386, 129508. Results are shown in FIG. 22. In the MEC3 and the MEC4 subjected to bioaugmentation with the strain 60473, it is confirmed that 40% or more of the biofilm on the surface of the anode electrode is Geobacter bacteria, and that the planted strain 60473 preferentially adheres to the surface of the anode electrode.

As shown in FIGS. 20 and 21, in the MEC3 and the MEC4 subjected to the bioaugmentation with the strain 60473, significantly larger currents are generated compared to the controls that are not subjected to the bioaugmentation (MEC1 and MEC2). Even after repeated electrolyte replacement, a maximum current density (peak current density) ($J_{max}$) achieved with the MEC3 and the MEC4 always reaches approximately 1.5 mA/cm$^2$, whereas with the MEC1 and the MEC2, the maximum current density is less than 0.04 mA/cm$^2$. The maximum current densities of the MEC3 and the MEC4 subjected to the bioaugmentation with the strain 60473 are unexpectedly high and comparable to those achieved in pure culture electrolysis cells with acetic acid as the substrate. These results suggest a possibility that, by the bioaugmentation with the strain 60473, performance of an electrolysis cell using a high molecular fermentation organic substance (HFO) such as starch as the substrate can be improved to a level same as performance of an electrolysis cell using acetic acid as the substrate.

In addition, in an electrolysis cell subjected to the bioaugmentation with the strain YM18, the maximum current density starts to decrease in a fourth cycle and a fifth cycle (Ochiai et al., Bioresource Technology, 2023, vol. 386, 129508). On the other hand, as shown in FIG. 21, it is confirmed that the maximum current densities of the MEC3 and the MEC4 do not change so much, and do not substantially decrease even during the operation of six cycles, and high current generating ability can be maintained for a long period of time. It is presumed that the generation of a constant current in the electrolysis cell subjected to the bioaugmentation with the strain 60473 is caused by high affinity to the graphite electrode of the strain 60473.

For the electrolysis performed in each electrolysis cell, the maximum current density [$J_{max}$ (mA/cm$^2$)], a daily hydrogen production rate (HPR: [generated hydrogen volume (L)]/[electrolytic solution volume (L) in the cell]/[Day]), and a removal rate of chemical oxygen demand (COD) [$COD_R$: (1 - [COD at the end of operation]/[COD at the start of operation]) × 100 (%)] were determined in the same manner as in Ochiai et al., Bioresource Technology, 2023, vol. 386, 129508. Results are shown in Table 2.

Table 2

| | $J_{max}$ (mA cm$^{-2}$) | HPR (L L$^{-1}$ D$^{-1}$) | $COD_R$ (%) |
|---|---|---|---|
| MFC1 | 0.020 ± 0.010 | N.D.[1] | 12 ± 3.3 |
| MFC2 | 0.021 ± 0.014 | N.D. | 7.3 ± 1.9 |
| MFC3 | 1.5 ± 0.30 | 0.54 ± 0.36 | 47 ± 2.1 |
| MFC4 | 1.5 ± 0.10 | 0.48 ± 0.37 | 54 ± 5.5 |

Hydrogen is generated only in the MEC3 and the MEC4 at a rate of about 0.5 L/L/Day. The $COD_R$ of the MEC3 and the MEC4 increases significantly more than that of the MEC1 and the MEC2. These results suggest that in the electrolysis cell subjected to the bioaugmentation with the strain 60473, most of the starch is decomposed along with current generation. In addition, Results in Table 2 show that the current generation (for example, a substance bound to the oxidation of acetic acid) is a rate-limiting step of starch decomposition in the electrolysis cell, and it is confirmed that enhancing this step by introducing a strong electrochemically active bacterium is a promising strategy of enhancing performance of the electrolysis cell for treating a high molecular fermentation organic substance.

Example 5

In order to evaluate usefulness of the strain 60473 in bioaugmentation of the microbial electrolysis cell, a three-electrode single-tank electrochemical cell was operated using a food waste as an anode substrate, the generated current was measured, and the power generation amount and the hydrogen production amount were examined (n = 2).

The food waste used was vegetable scraps from actual restaurants. Water was added to these crushed food wastes, and a mixture was allowed to stand for several days and then centrifuged to collect a supernatant. The supernatant was adjusted to pH 8.0 (COD value: about 55,000 ppm) and used as the anode substrate.

As the three-electrode single-tank electrochemical cell, the working electrode is graphite felt (6.00 cm$^2$, thickness 3 mm, manufactured by Nippon Carbon Co., Ltd.), the reference electrode is an Ag/AgCl electrode (RE-T8A, manufactured by EC Frontier), the counter electrode is platinum mesh (thickness 0.12 mm, manufactured by Nilaco Corporation), and the electrolyte is 50 mL. As the electrolyte, a solution obtained by adding, to 0.1 M PBS (pH 8.0), 12.3 g/L $Na_2HPO_4$, 1.9 g/L $KH_2PO_4$, 0.26 g/L $NH_4Cl$, a 10 mL/L modified Wallin's mineral solution (Modified Wolin's mineral solution, and Deutsche Sammlung von Mikroorganismen und Zellkulturen), and a 10 mL/L Wallin's vitamin solution (Wolin's vitamin solution, and Deutsche Sammlung von Mikroorganismen und Zellkulturen) is used.

Specifically, 5.5 mL of the anode substrate and 5 mL of anaerobic digestion sludge as an inoculum source were added to the electrolyte of the electrochemical cell, the cell was connected to a potentiostat (VMP3, manufactured by Biologic), a working electrode potential was set to -0.2 V vs. Ag/AgCl electrode, and current measurement was started.

During the operation, the electrolysis cell was intermittently replaced with a new electrolyte containing the anode substrate in order to add the anode substrate. A time from the time of electrolyte solution replacement to the next time of electrolyte solution replacement was defined as "one cycle", and the operation was continued until a sixth cycle (after performing the electrolyte solution replacement five times).

Two of the four electrolysis cells (60473_1, 60473_2) were added with the strain 60473 (bioaugmentation) such that the $OD_{600}$ of the strain 60473 in the cell was 0.04 after 4 hours when a current value of a first cycle became positive. The remaining two cells (NB_1, NB_2) were not ingested with the strain 60473 and continued to operate as controls without bioaugmentation.

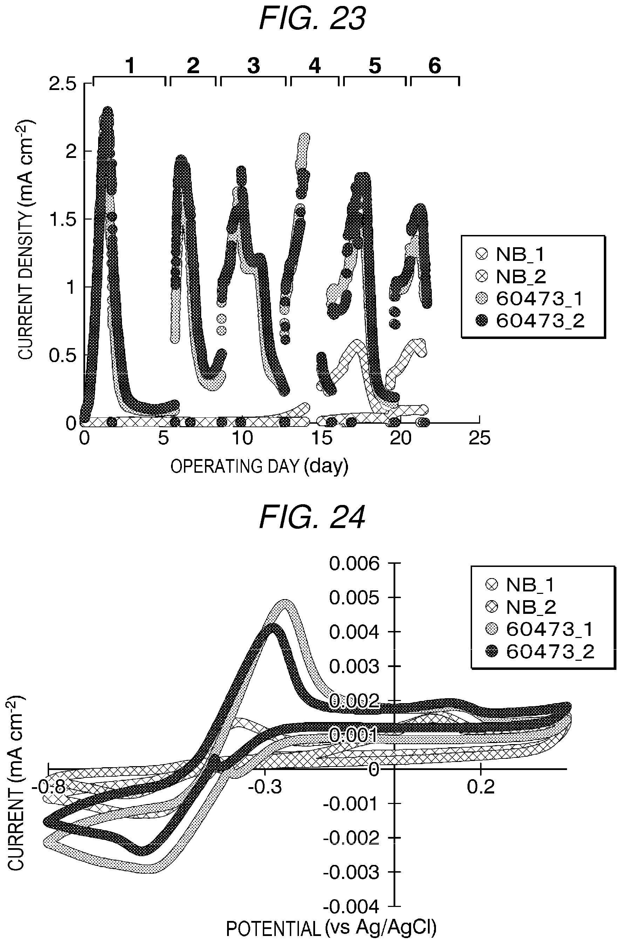

Results obtained by measuring the power generation amount (current density) of each cell over time are shown in FIG. 23. In FIG. 23, numbers at the top indicate "cycles". In the cells to which the strain 60473 was added (60473_1, 60473_2), the current density is significantly increased. Based on these results, it is confirmed that even in the case in which the food waste is used as the anode substrate, an effect of bioaugmentation using the strain 60473 is sufficiently obtained.

Further, for the electrolysis performed in each electrolysis cell, a removal rate of chemical oxygen demand (COD) [$COD_R$: (1 - [COD at the end of operation]/[COD at the start of operation]) $\times$ 100 (%)] and Coulombic efficiency (%) were determined. The Coulombic efficiency is a value expressed as a percentage of a ratio of discharge capacity during discharging to charge capacity, and the higher the Coulombic efficiency of a cell, the longer the life of the cell because the capacity gained during charging can be used for discharging without loss. The $COD_R$ was determined in the same manner as in Example 4. The Coulombic efficiency was determined using the following formula.

[Coulombic efficiency (%)] = [Coulombic equivalent value of integrated amount of generated current]/[Coulombic equivalent value of COD removal amount] $\times$ 100 (%)

Table 3 shows measurement results of the $COD_R$ of each electrolysis cell and the Coulombic efficiency at the sixth cycle. The cells to which the strain 60473 is added (60473_1, 60473_2) have a higher COD removal rate and higher Coulombic efficiency than the control cells (NB_1, NB_2). Based on these results, it is found that even when the food waste is used as the anode substrate, the COD can be more easily reduced and the life of the electrolysis cell can be further extended by the bioaugmentation using the strain 60473.

Table 3

| | | NB_1 | NB_2 | 60473_1 | 60473_2 |
|---|---|---|---|---|---|
| $COD_R$ (%) | First cycle | 29.5 | 34.1 | 28.9 | 31.2 |
| | Second cycle | 23.3 | 24.4 | 35.0 | 37.9 |
| | Third cycle | 36.6 | 34.9 | 47.4 | 50.0 |
| | Fourth cycle | 26.9 | 25.0 | 35.6 | 34.9 |
| | Fifth cycle | 33.5 | 36.0 | 29.4 | 41.2 |
| | Sixth cycle | 30.4 | 29.5 | 31.1 | 31.9 |
| | Final value | 30.3 $\pm$ 4.5 | | 35.2 $\pm$ 6.6 | |
| Coulombic efficiency of sixth cycle (%) | | 10.88 $\pm$ 4.47 | | 35.22 $\pm$ 1.28 | |

For the electrolysis performed in each electrolysis cell, the daily hydrogen production rate (HPR: [generated hydrogen volume (L)]/[electrolytic solution volume (L) in cell]/[Day]), a daily methane production rate (MPR: [generated methane volume (L)]/[electrolytic solution volume (L) in cell]/[Day]), and a methane generation amount (L/L/ day) were measured by gas chromatography. Results are shown in Table 4.

Table 4

| | | | NB_1 | NB_2 | 60473_1 | 60473_2 |
|---|---|---|---|---|---|---|
| HPR (L/L/day) | | First cycle | 0.00 | 0.00 | 0.51 | 0.54 |
| | | Second cycle | 0.00 | 0.00 | 0.40 | 0.28 |
| | | Third cycle | 0.00 | 0.00 | 0.16 | 0.03 |
| | | Fourth cycle | 0.00 | 0.00 | 0.02 | 0.01 |
| | | Fifth cycle | 0.00 | 0.00 | 0.00 | 0.02 |
| | | Sixth cycle | 0.00 | 0.00 | 0.00 | 0.00 |
| MPR (L/L/day) | | First cycle | 0.11 | 0.10 | 0.12 | 0.13 |
| | | Second cycle | 0.33 | 0.34 | 0.34 | - |
| | | Third cycle | 0.44 | 0.24 | 0.42 | 0.46 |
| | | Fourth cycle | 0.87 | 0.48 | 0.80 | 0.72 |
| | | Fifth cycle | 0.51 | 0.50 | 0.41 | 0.47 |
| | | Sixth cycle | 0.82 | 0.87 | 0.67 | 0.70 |
| Methane production amount (L/L/day) | | | 0.47 $\pm$ 0.26 | | 0.48 $\pm$ 0.22 | |

As shown in Table 4, in the cell to which the strain 60473 is added, generation of hydrogen is observed first, but almost no hydrogen is detected after the fourth cycle. This is presumed to be because the generated hydrogen is consumed by methane bacteria and the like. Meanwhile, no significant difference is found in the amount of methane generated between the cells to which the strain 60473 is added and the control cells.

Cyclic voltammetry (CV) is performed on each electrolysis cell after the sixth cycle. The CV is an electrochemical measurement method in which an electrode is immersed in a stationary sample solution and a potential is repeatedly scanned to measure a flowing current, which is expressed as a current-voltage curve. FIG. 24 shows CV results for each cell.

As shown in FIG. 24, a CV peak is larger in the cell to which the strain 60473 is added (60473_1, 60473_2) than in the control cells (NB_1, NB_2). This is presumed to be because an amount of electrochemically active bacteria adhering to the electrode is larger in the cell to which the strain 60473 is added.

Example 6

In order to evaluate usefulness of the strain 60473 in bioaugmentation of the microbial fuel cell (MFC), a one-tank air cathode MFC reactor was operated using a food waste as an anode substrate, the generated current was measured, and the power generation amount was examined (n = 2). The food waste prepared in the same manner as in Example 5 was used.

As the one-tank air cathode MFC reactor, a reactor volume is 85 mL, the anode electrode is graphite felt (12.56 $cm^2$, thickness 3 mm, manufactured by Nippon Carbon Co., Ltd.), the cathode electrode is an air cathode (25 $cm^2$, Yoshitsu et al., Microorganisms, 2023, vol. 11, 598.) coated with platinum catalyst (TEC10V20E, manufactured by Tanaka Kikinzoku Co., Ltd.) (0.64 $mg/cm^2$), and the electrolyte is 60 mL. As the electrolyte, an electrolyte having a composition same as that in Example 5 was used.

Specifically, 5.5 mL of an anode substrate and 6.5 mL of anaerobic digestion sludge used as an inoculum source were added to the electrolyte of the MFC reactor, and operation was started. An initial resistance was set to 10 k$\Omega$, and was lowered as needed when the pressure increased sufficiently. During the operation, the reactor was intermittently replaced with a new electrolyte containing the anode substrate in order to add the anode substrate. A time from the time of electrolyte solution replacement to the next time of electrolyte solution replacement was defined as "one cycle", and the operation was continued until a tenth cycle (after performing the electrolyte solution replacement nine times).

Two of the four MFC reactors (60473_1, 60473_2) were added with the strain 60473 (bioaugmentation) such that the $OD_{600}$ of the strain 60473 in the reactor was 0.04 after 4 hours when a current value of a first cycle became positive. The remaining two reactors (NB_1, NB_2) were not ingested with the strain 60473 and continued to operate as controls without bioaugmentation.

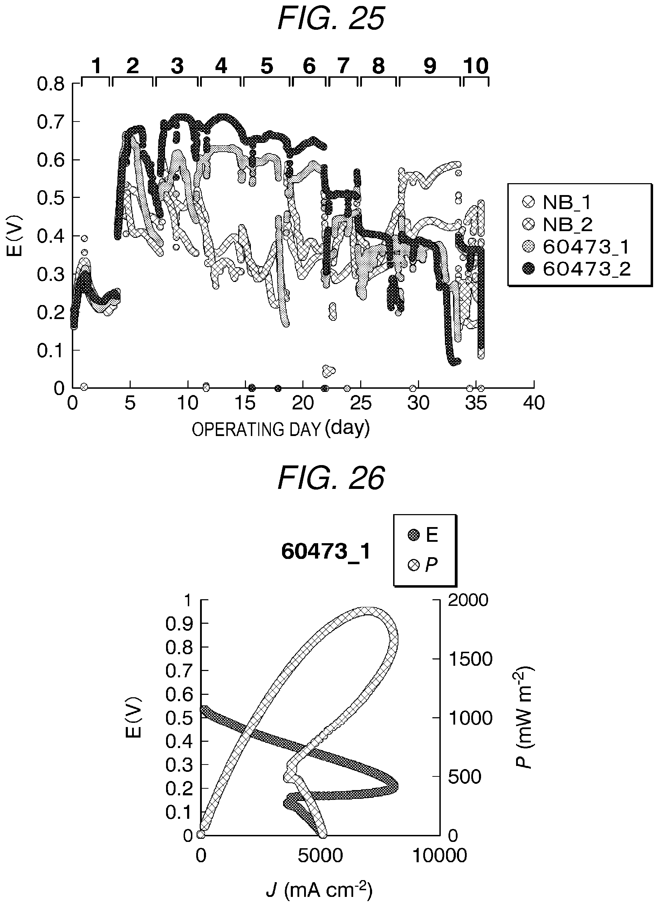

FIG. 25 shows results obtained by measuring a voltage (V) of each reactor over time. In FIG. 25, numbers at the top indicate "cycles". Table 5 shows the results obtained by measuring resistance values of each reactor over time for each cycle.

Table 5

| Number of cycles | Resistance (kΩ) | |
| --- | --- | --- |
| | NB_1 and NB_2 | 60473_1 and 60473_2 |
| 1 | 10.0 | 10.0 |
| 2 | 10.0 | 10.0 |
| 3 | 10.0 | 10.0 |
| 4 | 5.0 | 5.0 |
| 5 | 2.0 | 2.0 |
| 6 | 2.0 | 1.0 |
| 7 | 1.0 | 0.24 |
| 8 | 1.0 | 0.1 |
| 9 | 1.0 | 0.1 |
| 10 | 0.24 | 0.1 |

As shown in FIG. 25 and Table 5, in the reactors to which the strain 60473 is added (60473_1, 60473_2), a sufficient voltage (approximately 0.4 V or more) is generated even if the resistance is lowered. When a resistance is lowered, a large amount of current flows, and a large load is applied to the electrochemically active bacteria. Nevertheless, since a high voltage is maintained, it is confirmed that even with the MFC using the food waste as the anode substrate, an effect of bioaugmentation using the strain 60473 is sufficiently obtained.

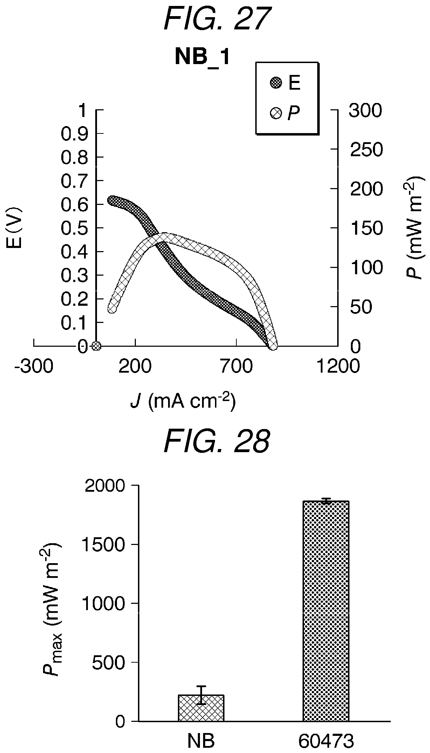

Results of polarization analysis for each reactor at the ninth cycle are shown in FIGS. 26 and 27. FIG. 26 shows polarization analysis results (n = 2) of the reactor into which the strain 60473 is introduced (60473_1, 60473_2). FIG. 27 shows polarization analysis results (n = 2) of the reactor into which the strain 60473 is not introduced (NB_1, NB_2). A maximum power density ($P_{max}$ mW/m$^2$) is calculated based on the polarization analysis results shown in FIGS. 26 and 27, and a comparison thereof is shown in FIG. 28. In the drawings, an error line indicates a standard deviation. As shown in FIG. 28, when the maximum power density obtained in the polarization analysis of the ninth cycle is compared in terms of the addition of the strain 60473, the output is significantly increased in the MFC to which the strain 60473 is added. Similar tendencies are observed in other cycles. These results indicate that even when the food waste is used as the anode substrate, cell performance can be further improved by performing the bioaugmentation using the strain 60473 on an existing microbial fuel cell.

Further, for the electrolysis performed in each reactor, a removal rate of chemical oxygen demand (COD) ($COD_R$) (%) and Coulombic efficiency (%) were determined in the same manner as in Example 5. The measurement results are shown in Table 6.

Table 6

| | | NB_1 | NB_2 | 60473_1 | 60473_2 |
| --- | --- | --- | --- | --- | --- |
| $COD_R$ (%) | First cycle | 59.0 | 70.5 | 58.4 | 52.8 |
| | Second cycle | 38.8 | 72.9 | 64.0 | 71.1 |
| | Third cycle | 55.9 | 66.6 | 66.2 | 63.9 |
| | Fourth cycle | 50.6 | 40.6 | 42.0 | 64.8 |
| | Fifth cycle | 38.6 | 52.1 | 55.7 | 13.3 |
| | Sixth cycle | 43.1 | 74.8 | 84.0 | 76.8 |
| | Seventh cycle | 34.4 | 46.6 | 58.7 | 66.6 |
| | Eighth cycle | 65.3 | 61.7 | 75.8 | 84.1 |
| | Ninth cycle | 67.5 | 80.0 | 82.3 | 83.8 |
| | Tenth cycle | 63.4 | 73.0 | 83.6 | 84.1 |
| | Seventh to ninth cycles | 61.5 ± 13.6 | | 77.4 ± 9.1 | |
| Coulombic efficiency of ninth cycle (%) | | 4.99 ± 1.81 | | 25.48 ± 2.18 | |

As shown in Table 6, the reactors to which the strain 60473 is added (60473_1, 60473_2) have a higher COD removal rate and higher Coulombic efficiency than the control reactors (NB_1, NB_2). Based on these results, it is found that even when the food waste is used as the anode substrate, the COD can be more easily reduced and the life of the MFC reactor can be further extended by the bioaugmentation using the strain 60473.

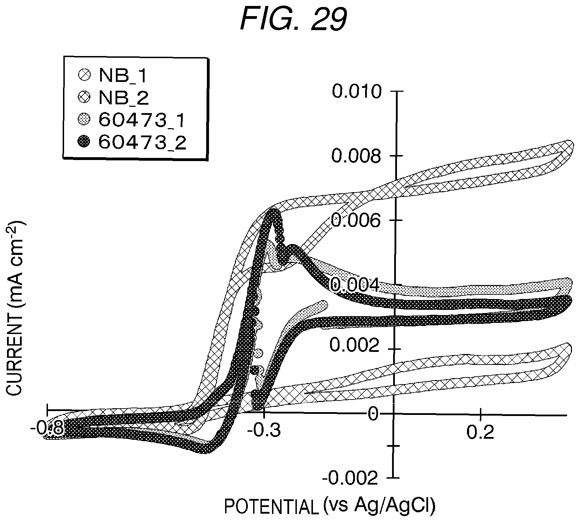

The CV was performed for each MFC reactor after the tenth cycle. FIG. 29 shows CV results for each reactor. As shown in FIG. 29, a CV peak is larger in the reactor to which the strain 60473 is added (60473_1, 60473_2) than in the control reactors (NB_1, NB_2). This is presumed to be because an amount of electrochemically active bacteria adhering to the electrode is larger in the cell to which the strain 60473 is added.

**Claims**

1. A microorganism comprising:
   a Geobacter sp. strain 60473, accession No.: NITE BP-03900.

2. The microorganism according to claim 1, which is used in a microbial fuel cell.

3. A microbial fuel cell composition comprising:

   a Geobacter sp. strain 60473, accession No.: NITE BP-03900; and
   an organic substance.

4. The microbial fuel cell composition according to claim 3, further comprising:
   an electrochemically active bacterium other than the Geobacter sp. strain 60473.

5. The microbial fuel cell composition according to claim 3, wherein
   at least a part of the organic substance is sludge.

6. A microbial fuel cell, wherein
   a Geobacter sp. strain 60473, accession No.: NITE BP-03900, is used to decompose an organic substance as a fuel.

7. A microbial fuel cell comprising:

   a liquid containing water containing an organic substance as a main component;
   a container configured to contain the liquid;
   a conductive anode electrode disposed in the liquid;
   a conductive cathode electrode disposed with at least one surface thereof in contact with the liquid;
   a resistor having one end connected to the anode electrode and the other end connected to the cathode electrode, through which electrons flow; and
   a Geobacter sp. strain 60473, accession No.: NITE BP-03900, which decomposes the organic substance contained in the liquid and discharges the electrons.

8. The microbial fuel cell according to claim 7, wherein

   the container is divided, by a membrane that allows protons to be selectively transmitted, into a tank in which the anode electrode is disposed and a tank in which the cathode electrode is disposed, and
   the organic substance and the Geobacter sp. strain 60473 are contained in the liquid contained in the tank in which the anode electrode is disposed.

9. The microbial fuel cell according to claim 7, wherein
   the cathode electrode is in contact with the liquid in the container on one surface and in contact with an atmosphere on the other surface.

10. A microbial fuel cell comprising:

    a container containing organic mud and a liquid containing water as a main component;
    a conductive anode electrode disposed in the organic mud;
    a conductive cathode electrode disposed within the liquid or on a liquid surface of the liquid;

a resistor having one end connected to the anode electrode and the other end connected to the cathode electrode, through which electrons flow; and

a Geobacter sp. strain 60473, accession No.: NITE BP-03900, contained in the organic mud.

11. A microbial fuel cell to be installed to purify organic mud at a bottom of a river or a lake, the microbial fuel cell comprising:

a conductive anode electrode disposed in organic mud;
a conductive cathode electrode disposed in water or on a water surface;
a resistor having one end connected to the anode electrode and the other end connected to the cathode electrode, through which electrons flow; and
a Geobacter sp. strain 60473, accession No.: NITE BP-03900, adhering to the anode electrode.

12. The microbial fuel cell according to claim 10, further comprising:
a supply tube configured to supply a substrate and/or the Geobacter sp. strain 60473 into the organic mud from outside.

13. The microbial fuel cell according to claim 12, wherein
the supply tube is branched into a plurality of tubes in a dispersed manner in a planar direction of the anode electrode so as to enable a supply into the liquid or the water.

14. The microbial fuel cell according to claim 7, wherein
the anode electrode is a conductive metal material having an internal electrical resistance lower than an internal electrical resistance of the cathode electrode.

15. The microbial fuel cell according to claim 7, wherein
a surface area of the cathode electrode is larger than a surface area of the anode electrode by 1.1 times or more.

16. The microbial fuel cell according to claim 10, wherein
the cathode electrode and the anode electrode are disposed in parallel, one end of the cathode electrode and one end of the anode electrode disposed on a same side as the one end are connected to a support body extending in a depth direction of the liquid or the water, the cathode electrode and the anode electrode are fixedly supported in an adjustable manner while maintaining an interval corresponding to a depth, and an installation fixing portion that protrudes in the depth direction is provided on a bottom side of the anode electrode.

17. A microbial electrolysis cell composition comprising:

a Geobacter sp. strain 60473, accession No.: NITE BP-03900; and
an organic substance.

18. The microbial electrolysis cell composition according to claim 17, further comprising:
an electrochemically active bacterium other than the Geobacter sp. strain 60473.

19. The microbial electrolysis cell composition according to claim 17, wherein
at least a part of the organic substance is sludge.

20. A microbial electrolysis cell, wherein
a Geobacter sp. strain 60473, accession No.: NITE BP-03900, is used to decompose an organic substance as a fuel.

21. A microbial electrolysis cell comprising:

a liquid containing water containing an organic substance as a main component;
a container configured to contain the liquid;
a conductive anode electrode disposed in the liquid;
a conductive cathode electrode disposed in contact with the liquid;
a potential control device having one end connected to the anode electrode and the other end connected to the cathode electrode, through which electrons flow; and
a Geobacter sp. strain 60473, accession No.: NITE BP-03900, which decomposes the organic substance contained in the liquid and discharges the electrons.

**22.** The microbial electrolysis cell according to claim 21, wherein

the container is divided, by a membrane that allows protons to be selectively transmitted, into a tank in which the anode electrode is disposed and a tank in which the cathode electrode is disposed, and
the organic substance and the Geobacter sp. strain 60473 are contained in the liquid contained in the tank in which the anode electrode is disposed.

**23.** A microbial electrolysis cell comprising:

a container containing organic mud and a liquid containing water as a main component;
a conductive anode electrode disposed in the liquid;
a conductive cathode electrode disposed in the liquid;
a potential control device having one end connected to the anode electrode and the other end connected to the cathode electrode, through which electrons flow; and
a Geobacter sp. strain 60473, accession No.: NITE BP-03900, contained in the organic mud.

**24.** The microbial electrolysis cell according to claim 23, further comprising:
a supply tube configured to supply a substrate and/or the Geobacter sp. strain 60473 into the liquid from outside.

**25.** The microbial electrolysis cell according to claim 24, wherein
the supply tube is branched into a plurality of tubes in a dispersed manner in the anode electrode or the cathode electrode so as to enable a supply into the liquid.

**26.** The microbial electrolysis cell according to claim 21, wherein
the anode electrode is a conductive material having an internal electrical resistance lower than an internal electrical resistance of the cathode electrode.

**27.** The microbial electrolysis cell according to claim 21, wherein
a surface area of the cathode electrode is larger than a surface area of the anode electrode by 1.1 times or more.

## FIG. 1

## FIG. 2

CELL INSTALLATION PLACE IS MOVABLE

## FIG. 3

## FIG. 4

## FIG. 5

## FIG. 6

## FIG. 7

## FIG. 8

## FIG. 9

## FIG. 10

## FIG. 11

## FIG. 12

## FIG. 13

Legend:
- ⊘ STRAIN PCA
- ⊘ STRAIN KN400
- ⊗ STRAIN YM18
- ● STRAIN 60473

## FIG. 14

## FIG. 15

## FIG. 16

## FIG. 17

## FIG. 18

# FIG. 19

# FIG. 20

## FIG. 21

## FIG. 22

## FIG. 23

## FIG. 24

## FIG. 25

## FIG. 26

**60473_1**

## FIG. 27

## FIG. 28

## FIG. 29

Europäisches
Patentamt

European
Patent Office

Office européen
des brevets

# EUROPEAN SEARCH REPORT

**Application Number**

EP 24 19 7181

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | FUJIKAWA TAKASHI ET AL: "Unexpected genomic features of high current density-producing Geobacter sulfurreducens strain YM18", FEMS MICROBIOLOGY LETTERS, vol. 368, no. 17, 2 September 2021 (2021-09-02), XP093248549, ISSN: 1574-6968, DOI: 10.1093/femsle/fnab119 Retrieved from the Internet: URL:http://academic.oup.com/femsle/article-pdf/368/17/fnab119/40857471/fnab119.pdf> * the whole document * | 1-27 | INV. C12N1/20 C12R1/01 H01M8/16 |
| A,D | OCHIAI ITTA ET AL: "Bioaugmentation of microbial electrolysis cells with Geobacter sulfurreducens YM18 for enhanced hydrogen production from starch", BIORESOURCE TECHNOLOGY, vol. 386, 17 July 2023 (2023-07-17), page 129508, XP093248459, AMSTERDAM, NL ISSN: 0960-8524, DOI: 10.1016/j.biortech.2023.129508 Retrieved from the Internet: URL:https://pdf.sciencedirectassets.com/271433/1-s2.0-S0960852423X00170/1-s2.0-S0960852423009367/main.pdf?hash=526302557e171052f48051f1998d003d772f3e96264d42ac9497980156f0e8c4&host=68042c943591013ac2b2430a89b270f6af2c76d8dfd086a07176afe7c76c2c61&pii=S0960852423009367&tid=spdf-780a8af2-343e-45f5-b9ec-361> * the whole document * | 1-27 | TECHNICAL FIELDS SEARCHED (IPC) C12N C12R H01M |

-/--

The present search report has been drawn up for all claims

| Place of search | Date of completion of the search | Examiner |
|---|---|---|
| The Hague | 10 February 2025 | Oderwald, Harald |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or after the filing date
D : document cited in the application
L : document cited for other reasons

                                                          

& : member of the same patent family, corresponding document

EPO FORM 1503 03.82 (P04C01)

Europäisches
Patentamt

European
Patent Office

Office européen
des brevets

# EUROPEAN SEARCH REPORT

Application Number

EP 24 19 7181

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | YI H ET AL: "Selection of a variant of Geobacter sulfurreducens with enhanced capacity for current production in microbial fuel cells", BIOSENSORS AND BIOELECTRONICS, ELSEVIER SCIENCE LTD, UK, AMSTERDAM , NL, vol. 24, no. 12, 15 August 2009 (2009-08-15), pages 3498-3503, XP026458650, ISSN: 0956-5663 [retrieved on 2009-05-14] * the whole document * | 1-27 | |
| A | FUJIKAWA TAKASHI ET AL: "Complete Genome Sequence of High Current-Producing Geobacter sulfurreducens Strain YM35, Isolated from River Sediment in Japan", MICROBIOLOGY RESOURCE ANNOUNCEMENTS, vol. 10, no. 33, 19 August 2021 (2021-08-19), XP093248552, ISSN: 2576-098X, DOI: 10.1128/MRA.00539-21 Retrieved from the Internet: URL:https://journals.asm.org/doi/pdf/10.11 28/MRA.00539-21> | 1-27 | |
| A | DAN SUN ET AL: "Geobacter sp. SD-1 with enhanced electrochemical activity in high-salt concentration solutions", ENVIRONMENTAL MICROBIOLOGY REPORTS, WILEY-BLACKWELL PUBLISHING, GB, vol. 6, no. 6, 16 July 2014 (2014-07-16), pages 723-729, XP072392467, ISSN: 1758-2229, DOI: 10.1111/1758-2229.12193 * the whole document * | 1-27 | TECHNICAL FIELDS SEARCHED (IPC) |

-/--

The present search report has been drawn up for all claims

| Place of search | Date of completion of the search | Examiner |
|---|---|---|
| The Hague | 10 February 2025 | Oderwald, Harald |

EPO FORM 1503 03.82 (P04C01)

Europäisches
Patentamt

European
Patent Office

Office européen
des brevets

# EUROPEAN SEARCH REPORT

Application Number

EP 24 19 7181

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | WANG ZHIGAO ET AL: "Enhancing electrical outputs of the fuel cells with Geobacter sulferreducens by overexpressing nanowire proteins", MICROBIAL BIOTECHNOLOGY, vol. 16, no. 3, 3 August 2022 (2022-08-03) , pages 534-545, XP093248183, GB ISSN: 1751-7915, DOI: 10.1111/1751-7915.14128 Retrieved from the Internet: URL:https://onlinelibrary.wiley.com/doi/full-xml/10.1111/1751-7915.14128> * the whole document * | 1-27 | |

- - - - -

TECHNICAL FIELDS
SEARCHED      (IPC)

The present search report has been drawn up for all claims

| Place of search | Date of completion of the search | Examiner |
|---|---|---|
| The Hague | 10 February 2025 | Oderwald, Harald |

CATEGORY OF CITED DOCUMENTS

X : particularly relevant if taken alone
Y : particularly relevant if combined with another
　document of the same category
A : technological background
O : non-written disclosure
P : intermediate document

T : theory or principle underlying the invention
E : earlier patent document, but published on, or
　after the filing date
D : document cited in the application
L : document cited for other reasons

 

& : member of the same patent family, corresponding
　document

EPO FORM 1503 03.82 (P04C01)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 2023142590 A
- JP 2023199255 A
- JP 2024006867 A
- JP 2010218690 A
- JP 2020014418 A

### Non-patent literature cited in the description

- Basics and Applications of Microbial Fuel Cells. **SHOHEI YAMADA** ; **KAZUYA WATANABE**. Fuel Cells. Fuel Cell Development Information Center, 2020, vol. 20, 33-38
- Hydrogen Production Using Microbial Electrolysis Tank. **ICHITA OCHIAI et al.** Power Generation and Hydrogen Production Using Microorganisms. CMC Publishing Co., Ltd., 2021, 201-206
- **YAMADA et al.** *Frontiers in Chemistry*, 2022, vol. 9
- **LU** ; **REN**. *Bioresource Technology*, 2016, vol. 215, 254-264
- **YANUKA-GOLUB et al.** *mBio*, 2021, vol. 12, e03629-20
- **OCHIAI et al.** *Bioresource Technology*, 2023, vol. 386, 129508
- **FUJIKAWA et al.** *FEMS Microbiology Letters*, 2021, vol. 368 (17), fnab119
- **YOSHITSU et al.** *Microorganisms*, 2023, vol. 11, 598